(19) Europäisches Patentamt
European Patent Office
Office européen des brevets

(11) Veröffentlichungsnummer: **0 304 624 A2**

(12) **EUROPÄISCHE PATENTANMELDUNG**

(21) Anmeldenummer: **88111717.0**

(22) Anmeldetag: **20.07.88**

(51) Int. Cl.⁴: **C07D 401/04 , C07D 495/04 , A61K 31/44 , //C07D401/12, C07D235/28**

Patentansprüche für folgende Vertragsstaaten: ES + GR.

(30) Priorität: **29.07.87 CH 2906/87**

(43) Veröffentlichungstag der Anmeldung: **01.03.89 Patentblatt 89/09**

(84) Benannte Vertragsstaaten: **AT BE CH DE ES FR GB GR IT LI LU NL SE**

(71) Anmelder: **F. HOFFMANN-LA ROCHE & CO. Aktiengesellschaft**

**CH-4002 Basel(CH)**

(72) Erfinder: **Fischli, Albert, Prof. Dr.**
**Im Wenkenberg 20**
**CH-4125 Riehen(CH)**
Erfinder: **Krasso, Anna, Dr.**
**Rütimeyerstrasse 35**
**CH-4054 Basel(CH)**
Erfinder: **Szente, André, Dr.**
**Baselstrasse 70**
**CH-4125 Riehen(CH)**

(74) Vertreter: **Lederer, Franz, Dr. et al**
**Van der Werth, Lederer & Riederer**
**Patentanwälte Lucile-Grahn-Strasse 22**
**D-8000 München 80(DE)**

(54) **Benzimidazol-2-yl-pyridiniumverbindungen.**

(57) Benzimidazol-2-yl-pyridiniumverbindungen der allgemeinen Formel

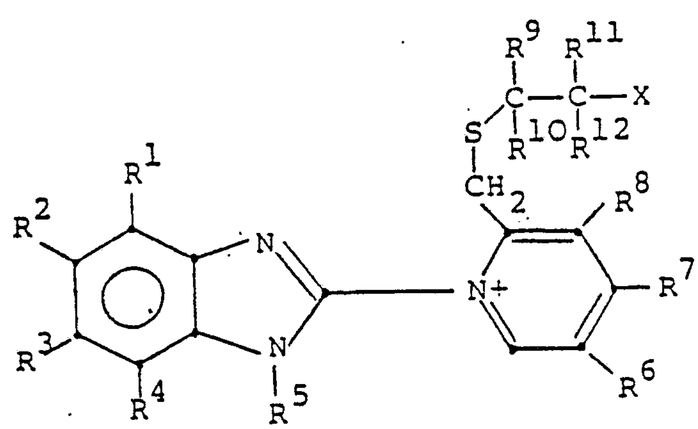

I

worin
- $R^1$, $R^2$, $R^3$ und $R^4$ je Wasserstoff, Fluor, Chlor, Trifluormethyl, Cyano oder einen Rest der Formel

EP 0 304 624 A2

$-COOR^{13}$ , $-CONR^{14}R^{15}$ , $-SOR^{16}$ oder $-SO_2R^{16}$

(a) (b) (c) (d)

oder zwei benachbarte dieser Substituenten zusammen mit den Kohlenstoffatomen, an welchen sie gebunden sind, einen mindestens eines der Strukturelemente

$-CO-$ , $-COO-$ , $-CON(R^{14})-$ , $-SO-$ oder $-SO_2-$

(f) (g) (h) (i) (j)

enthaltenden 5-, 6- oder 7-gliedrigen Ring bedeuten, mit der Massgabe, dass von den Symbolen $R^1$, $R^2$, $R^3$ und $R^4$ mindestens eines, höchstens aber drei Wasserstoff bedeuten;
- $R^5$ Wasserstoff oder eine negative Ladung bedeutet;
- $R^6$ und $R^8$ je Wasserstoff oder niederes Alkyl bedeuten;
- $R^7$ niederes Alkoxy bedeutet;
- $R^9$ und $R^{10}$ je Wasserstoff oder niederes Alkyl und $R^{11}$ und $R^{12}$ je niederes Alkyl bedeuten; oder zwei der Substituenten $R^9$ und $R^{10}$ oder $R^9$ und $R^{11}$ oder $R^{11}$ und $R^{12}$ zusammen mit dem oder den Kohlenstoffatom(en), an welche(s) sie gebunden sind, einen 5-, 6- oder 7-gliedrigen carbocyclischen Ring und die beiden übrigen der Substituenten $R^9$, $R^{10}$, $R^{11}$ und $R^{12}$ je Wasserstoff oder niederes Alkyl bedeuten; oder $R^9$ zusammen mit X eine zusätzliche Kohlenstoff-Kohlenstoff-Bindung, $R^{10}$ Wasserstoff oder niederes Alkyl und $R^{11}$ und $R^{12}$ je niederes Alkyl bedeuten;
- $R^{13}$ niederes Alkyl bedeutet;
- $R^{14}$ und $R^{15}$ je Wasserstoff oder niederes Alkyl oder zusammen mit dem Stickstoffatom einen 5-, 6- oder 7-gliedrigen gesättigten heterocyclischen Ring bedeuten;
- $R^{16}$ niederes Alkyl bedeutet;
- X Chlor, Brom oder einen Rest der Formel $-OR^{17}$ bedeutet oder, wie schon erwähnt, zusammen mit $R^9$ eine zusätzliche Kohlenstoff-Kohlenstoff-Bindung bedeutet;
- $R^{17}$ Wasserstoff, Acyl oder einen Rest der Formel

$-CH(R^{18})R^{19}$

(k)

bedeutet;
- $R^{18}$ Wasserstoff oder niederes Alkyl bedeutet; und
- $R^{19}$ Wasserstoff, niederes Alkyl, niederes Alkenyl, niederes Hydroxyalkyl, niederes Alkoxy-niederes-Alkyl, Hydroxy-niederes-Alkoxy-niederes-Alkyl, niederes Alkoxy-niederes-Alkoxy-niederes-Alkyl, Hydroxy-niederes-Alkoxy-niederes-Alkoxy-niederes-Alkyl, niederes Alkoxy-niederes-Alkoxy-niederes-Alkoxy-niederes-Alkyl, Hydroxy-niederes-Alkoxy-niederes-Alkoxy-niederes-Alkoxy-niederes-Alkyl, Acyloxy-niederes-Alkyl oder Acyloxy-niederes-Alkoxy-niederes-Alkyl bedeutet;

wobei das Molekül gesamthaft ungeladen oder einfach positiv geladen ist und wobei im letzteren Fall ein externes Anion vorliegt, besitzen mangesäuresekretionshemmende und/oder mukosaprotektive Eigenschaften, so dass sie zur Bekämpfung oder Verhütung von Krankheiten des Magen-Darm-Trakts verwendet werden können, insbesondere gegen Ulcus ventriculi und duodeni.

## Benzimidazol-2-yl-pyridiniumverbindungen

Die vorliegende Erfindung betrifft Benzimidazol-Derivate. Im speziellen betrifft sie Benzimidazol-2-yl-pyridiniumverbindungen der allgemeinen Formel

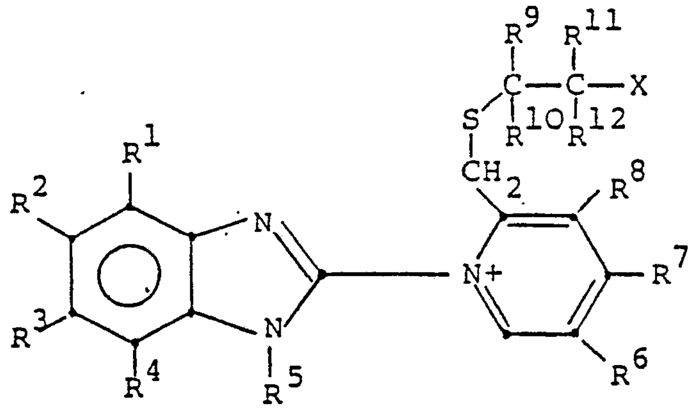

I

worin

- $R^1$, $R^2$, $R^3$ und $R^4$ je Wasserstoff, Fluor, Chlor, Trifluormethyl, Cyano oder einen Rest der Formel

$-COOR^{13}$ (a), $-CONR^{14}R^{15}$ (b), $-SOR^{16}$ (c) oder $-SO_2R^{16}$ (d)

oder zwei benachbarte dieser Substituenten zusammen mit den Kohlenstoffatomen, an welchen sie gebunden sind, einen mindestens eines der Strukturelemente

$-CO-$ (f), $-COO-$ (g), $-CON(R^{14})-$ (h), $-SO-$ (i) oder $-SO_2-$ (j)

enthaltenden 5-, 6- oder 7-gliedrigen Ring bedeuten, mit der Massgabe, dass von den Symbolen $R^1$, $R^2$, $R^3$ und $R^4$ mindestens eines, höchstens aber drei Wasserstoff bedeuten;

- $R^5$ Wasserstoff oder eine negative Ladung bedeutet;
- $R^6$ und $R^8$ je Wasserstoff oder niederes Alkyl bedeuten;
- $R^7$ niederes Alkoxy bedeutet;
- $R^9$ und $R^{10}$ je Wasserstoff oder niederes Alkyl und $R^{11}$ und $R^{12}$ je niederes Alkyl bedeuten; oder zwei der Substituenten $R^9$ und $R^{10}$ oder $R^9$ und $R^{11}$ oder $R^{11}$ und $R^{12}$ zuammen mit dem oder den Kohlenstoffatom(en), an welche(s) sie gebunden sind, einen 5-, 6- oder 7-gliedrigen carbocyclischen Ring und die beiden übrigen der Substituenten $R^9$, $R^{10}$, $R^{11}$ und $R^{12}$ je Wasserstoff oder niederes Alkyl bedeuten; oder $R^9$ zusammen mit X eine zusätzliche Kohlenstoff-Kohlenstoff-Bindung, $R^{10}$ Wasserstoff oder niederes Alkyl und $R^{11}$ und $R^{12}$ je niederes Alkyl bedeuten;
- $R^{13}$ niederes Alkyl bedeutet;
- $R^{14}$ und $R^{15}$ je Wasserstoff oder niederes Alkyl oder zusammen mit dem Stickstoffatom einen 5-, 6- oder 7-gliedrigen gesättigten heterocyclischen Ring bedeuten;
- $R^{16}$ niederes Alkyl bedeutet;
- X Chlor, Brom oder einen Rest der Formel $-OR^{17}$ bedeutet oder, wie schon erwähnt, zusammen mit $R^9$

eine zusätzliche Kohlenstoff-Kohlenstoff-Bindung bedeutet;
- $R^{17}$ Wasserstoff, Acyl oder einen Rest der Formel

$$-CH(R^{18})R^{19}$$

(k)

bedeutet;
- $R^{18}$ Wasserstoff oder niederes Alkyl bedeutet; und
- $R^{19}$ Wasserstoff, niederes Alkyl, niederes Alkenyl, niederes Hydroxyalkyl, niederes Alkoxy-niederes-Alkyl, Hydroxy-niederes-Alkoxy-niederes-Alkyl, niederes Alkoxy-niederes-Alkoxy-niederes Alkyl, Hydroxy-niederes-Alkoxy-niederes-Alkoxy-niederes-Alkyl, niederes Alkoxy-niederes-Alkoxy-niederes-Alkoxy-niederes-Alkyl, Hydroxy-niederes-Alkoxy-niederes-Alkoxy-niederes-Alkoxy-niederes-Alkyl, Acyloxy-niederes-Alkyl oder Acyloxy-niederes-Alkoxy-niederes-Alkyl bedeutet;

wobei das Molekül gesamthaft ungeladen oder einfach positiv geladen ist und wobei im letzteren Fall ein externes Anion vorliegt.

Diese Verbindungen sind neu und es hat sich gezeigt, dass sie wertvolle pharmakodynamische Eigenschaften besitzen, nämlich magensäuresekretions-hemmende und/oder mukosaprotektive Eigenschaften (speziell gegen Indomethacin-induzierte Läsionen), sodass sie zur Bekämpfung oder Verhütung von Krankheiten des Magen-Darm-Trakts verwendet werden können, insbesondere gegen Ulcus ventriculi und duodeni.

Gegenstand der vorliegenden Erfindung sind die eingangs definierten neuen Verbindungen als solche und als therapeutische Wirkstoffe, die Herstellung dieser neuen Verbindungen, Arzneimittel, enthaltend eine solche Verbindung, die Herstellung solcher Arzneimittel, sowie die Verwendung der eingangs definierten Verbindungen bei der Bekämpfung bzw. Verhütung von Krankheiten, insbesondere bei der Bekämpfung bzw. Verhütung von Ulcus ventriculi und duodeni bzw. die Verwendung der eingangs definierten Verbindungen zur Herstellung von Arzneimitteln für die Bekämpfung bzw. Verhütung von Ulcus ventriculi und duodeni.

Wenn das Molekül gesamthaft ungeladen ist, dann liegen die Benzimidazol-2-yl-pyridiniumverbindungen der allgemeinen Formel I in Form innerer Salze vor. Dies ist dann der Fall, wenn $R^5$ eine negative Ladung bedeutet.

Der Ausdruck "nieder" bezeichnet Reste oder Verbindungen mit biz zu 7, vorzugsweise bis zu 4 Kohlenstoffatomen.

Der Ausdruck "Alkyl" bezeichnet geradkettige oder verzweigte gesättigte Kohlenwasserstoffreste, wie Methyl, Aethyl, n-Propyl, i-Propyl, sec-Butyl, t-Butyl und dgl.

Der Ausdruck "Alkoxy"bezeichnet über ein Sauerstoffatom gebundene Alkylgruppen im Sinne der vorstehenden Definition.

Der Ausdruck "Acyl" bezeichnet Reste, die sich von organischen Säuren, insbesondere Carbonsäuren, durch Elimination der Hydroxygruppe ableiten, und er umfasst in erster Linie niedere Alkanoylreste, wie Acetyl, Propionyl und dgl.

Der 5-, 6- oder 7-gliedrige Ring, den zwei benachbarte der Substituenten $R^1$, $R^2$, $R^3$ und $R^4$ gemeinsam und zusammen mit den Kohlenstoffatomen, an welche sie gebunden sind, bilden können, kann heterocyclisch oder carbocyclisch sein, er kann gegebenenfalls eine oder mehrere zusätzliche Doppelbindungen enthalten, wobei er aromatisch oder nicht aromatisch sein kann, und er kann substituiert oder unsubstituiert sein; als Substituenten kommen niederes Alkyl, insbesondere Methyl, Oxo, oder dgl. in Betracht.

Zweckmässigerweise bedeuten $R^1$ und $R^4$ je Wasserstoff und entweder $R^2$ und $R^3$ je Fluor oder je Chlor oder zusammen einen Rest der Formel $-C(CH_3)_2-CO-C(CH_3)_2-$oder $R^2$ Fluor oder Trifluormethyl und $R^3$ Wasserstoff. Vorzugsweise bedeuten $R^2$ Trifluormethyl und $R^1$, $R^3$ und $R^4$ je Wasserstoff.

Weiterhin bedeuten zweckmässigerweise $R^6$ Wasserstoff oder Methyl, $R^7$ Methoxy oder Aethoxy und $R^8$ Methyl; dabei ist für $R^7$ die Bedeutung Methoxy bevorzugt.

Schliesslich bedeuten zweckmässigerweise entweder $R^9$ und $R^{10}$ je Wasserstoff, $R^{11}$ und $R^{12}$ je Methyl und X Chlor, Hydroxy, Methoxy, Aethoxy, Propoxy, Butoxy, Acetoxy, 2-Hydroxyäthoxy, 2-Methoxyäthoxy, 2-(2-Hydroxyäthoxy)äthoxy, 2-(2-Methoxyäthoxy)äthoxy, 2-[2-(2-Hydroxyäthoxy)äthoxy]äthoxy oder 2-[2-(2-Methoxyäthoxy)äthoxy]äthoxy; oder $R^9$ zusammen mit X eine zusätzliche Kohlenstoff-Kohlenstoff-Bindung, $R^{10}$ Wasserstoff und $R^{11}$ und $R^{12}$ je Methyl; oder $R^9$ und $R^{11}$ zusammen Tetramethylen, $R^{10}$ und $R^{12}$ je

Wasserstoff und X Methoxy. Vorzugsweise bedeuten $R^9$ und $R^{10}$ je Wasserstoff, $R^{11}$ und $R^{12}$ je Methyl und X Chlor, Hydroxy, Methoxy, Aethoxy, Propoxy, Acetoxy, 2-Hydroxyäthoxy oder 2-Methoxyäthoxy; oder $R^9$ zusammen mit X eine zusätzliche Kohlenstoff-Kohlenstoff-Bindung, $R^{10}$ Wasserstoff und $R^{11}$ und $R^{12}$ je Methyl. In einer besonders bevorzugten Ausführungsform bedeuten $R^9$ und $R^{10}$ je Wasserstoff, $R^{11}$ und $R^{12}$ je Methyl und X Aethoxy, Acetoxy, 2-Hydroxyäthoxy oder 2-Methoxyäthoxy.

Eine ganz besonders bevorzugte Verbindung der Formel I ist:
2-[[[2-(2-Hydroxyäthoxy)-2-methylpropyl]thio]methyl]-4-methoxy-3-methyl-1-[5-(trifluormethyl)-2-benzimidazolyl]pyridinium-methansulfonat.

Weitere besonders bevorzugte Verbindungen der Formel I sind:
Intramolekular deprotonisiertes 2-[[[2-(2-Hydroxyäthoxy)-2-methylpropyl]thio]methyl]-4-methoxy-3-methyl-1-[5-(trifluormethyl)-2-benzimidazolyl]pyridiniumkation:
4-Methoxy-2-[[[2-(2-methoxyäthoxy)-2-methylpropyl]thio]methyl]-3-methyl-1-[5-(trifluormethyl)-2-benzimidazolyl]pyridinium-methansulfonat;
intramolekular deprotonisiertes 4-Methoxy-2-[[[2-(2-methoxyäthoxy)-2-methylpropyl]thio]methyl]-3-methyl-1-[5-(trifluormethyl)-2-benzimidazolyl]pyridiniumkation;
2-[[(2-Acetoxy-2-methylpropyl)thio]methyl]-4-methoxy-3-methyl-1-[5-(trifluormethyl)-2-benzimidazolyl]-pyridinium-methansulfonat;
2-[[(2-Acetoxy-2-methylpropyl)thio]methyl]-4-methoxy-3-methyl-1-[5-(trifluormethyl)-2-benzimidazolyl]-pyridiniumchlorid;
intramolekular deprotonisiertes 2-[[(2-Acetoxy-2-methylpropyl)thio]methyl]-4-methoxy-3-methyl-1-[5-(trifluormethyl)-2-benzimidazolyl]pyridiniumkation;
2-[[(2-Aethoxy-2-methylpropyl)thio]methyl]-4-methoxy-3-methyl-1-[5-(trifluormethyl)-2-benzimidazolyl]-pyridinium-methansulfonat;
2-[[(2-Aethoxy-2-methylpropyl)thio]methyl]-4-methoxy-3-methyl-1-[5-(trifluormethyl)-2-benzimidazolyl]-pyridiniumchlorid; und
intramolekular deprotonisiertes 2-[[(2-Aethoxy-2-methylpropyl)thio]methyl]-4-methoxy-3-methyl-1-[5-(trifluormethyl)-2-benzimidazolyl]pyridiniumkation.

Ebenfalls bevorzugte Verbindungen der Formel I sind beispielsweise:
Intramolekular deprotonisiertes 2-[[(2-Chlor-2-methylpropyl)thio]methyl]-4-methoxy-3-methyl-1-[5-(trifluormethyl)-2-benzimidazolyl]pyridiniumkation;
2-[[(2-Chlor-2-methylpropyl)thio]methyl]-4-methoxy-3-methyl-1-[5-(trifluormethyl)-2-benzimidazolyl]-pyridiniumchlorid;
intramolekular deprotonisiertes 2-[[(2-Hydroxy-2-methylpropyl)thio]methyl]-4-methoxy-3-methyl-1-[5-(trifluormethyl)-2-benzimidazolyl]pyridiniumkation;
intramolekular deprotonisiertes 4-Methoxy-2-[[(2-methoxy-2-methylpropyl)thio]methyl]-3-methyl-1-[5-(trifluormethyl)-2-benzimidazolyl]pyridiniumkation;
intramolekular deprotonisiertes 4-Methoxy-3-methyl-2-[[(2-methylpropenyl)thio]methyl]-1-[5-)trifluormethyl)-2-benzimidazolyl]pyridiniumkation;
intramolekular deprotonisiertes 4-Methoxy-3-methyl-2-[[(2-methyl-2-propoxypropyl)thio]methyl]-1-[5-(trifluormethyl)-2-benzimidazolyl]pyridiniumkation;
4-Methoxy-3-methyl-2-[[(2-methylpropenyl)thio]methyl]-1-[5-(trifluormethyl)-2-benzimidazolyl]-pyridiniumchlorid; und
intramolekular deprotonisiertes 2-[[(2-Hydroxy-2-methylpropyl)thio]methyl]-4-methoxy-3,5-dimethyl-1-[5-(trifluormethyl)-2-benzimidazolyl]pyridniumkation.

Die Verbindungen der Formel I können erfindungsgemäss dadurch hergestellt werden, dass man

a) eine Verbindung der allgemeinen Formel

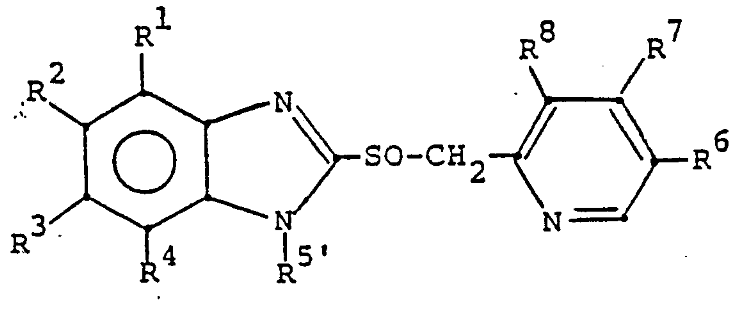

II

worin $R^1$, $R^2$, $R^3$, $R^4$, $R^6$, $R^7$ und $R^8$ obige Bedeutung besitzen und $R^{5'}$ Wasserstoff bedeutet,

unter sauren Bedingungen mit einer Verbindung der allgemeinen Formel

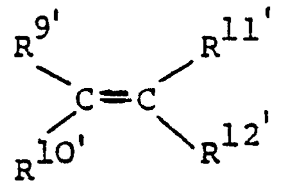

III

worin $R^{9'}$ und $R^{10'}$ je Waserstoff oder niederes Alkyl und $R^{11'}$ und $R^{12'}$ je niederes Alkyl bedeuten, oder zwei der Substituenten $R^{9'}$ und $R^{10'}$ oder $R^{9'}$ und $R^{11'}$ oder $R^{11'}$ und $R^{12'}$ zusammen mit dem oder den Kohlenstoffatom(en), an welche(s) sie gebunden sind, einen 5-, 6- oder 7-gliedrigen carbocyclischen Ring und die beiden übrigen der Substituenten $R^{9'}$, $R^{10'}$ $R^{11'}$ und $R^{12'}$ je Wasserstoff oder niederes Alkyl bedeuten,
und einer Verbindung der allgemeinen Formel

$HX'$ IV

worin $X'$ Chlor, Brom oder einen Rest der Formel $-OR^{17}$ und $OR^{17}$ Wasserstoff oder einen Rest der obigen Formel (k) bedeuten,
umsetzt; oder

b) eine Verbindung der Formel I, worin X Chlor oder Brom bedeutet, mit einer Verbindung der allgemeinen Formel

$HOR^{17'}$ V

worin $R^{17'}$ Wasserstoff oder einen Rest der obigen Formel (k) bedeutet,
umsetzt; oder

c) eine Verbindung der Formel I, worin X einen Rest der Formel $-OR^{17}$ und $R^{17}$ Wasserstoff bedeuten, dehydratisiert; oder

d) eine Verbindung der Formel I, worin X einen Rest der Formel $-OR^{17}$, $R^{17}$ Wasserstoff oder einen Rest der obigen Formel (k) und $R^{19}$ niederes Hydroxyalkyl oder Hydroxy-niederes Alkoxy-niederes-Alkyl bedeuten, acyliert; worauf man das erhaltene Produkt als Salz oder inneres Salz isoliert und erwünschtenfalls ein inneres Salz in ein pharmazeutisch annehmbares Salz überführt.

Gemäss Aspekt a) des erfindungsgemässen Verfahrens erhält man Verbindungen der Formel I, worin entweder $R^9$ und $R^{10}$ je Wasserstoff oder niederes Alkyl und $R^{11}$ und $R^{12}$ je niederes Alkyl bedeuten oder worin zwei der Substituenten $R^9$ und $R^{10}$ oder $R^9$ und $R^{11}$ oder $R^{11}$ und $R^{12}$ zusammen mit dem oder den Kohlenstoffatom(en), an welche(s) sie gebunden sind, einen 5-, 6- oder 7-gliedrigen carbocyclischen Ring und die beiden übrigen der Substituenten $R^9$, $R^{10}$, $R^{11}$ und $R^{12}$ je Wasserstoff oder niederes Alkyl bedeuten und worin X Chlor, Brom oder einen Rest der Formel $-OR^{17}$ und $R^{17}$ Wasserstoff oder einen Rest der Formel (k) bedeuten. Als Komponente der Formel III verwendet man Verbindungen, wie Isobutylen, Cyclohexen oder dgl., und als Komponente der Formel IV Chlorwasserstoff, Bromwasserstoff. Wasser oder einen Alkohol der Formel

$HO-CH(R^{18})R^{19}$ VI

worin $R^{18}$ und $R^{19}$ die eingangs erwähnte Bedeutung besitzen.

Verwendet man als Komponente der Formel IV Chlorwasserstoff oder Bromwasserstoff, so erhält man eine entsprechende Verbindung der Formel I, worin X Chlor bzw. Brom bedeutet. Hierbei wird das Ausgangsprodukt der Formel II in Gegenwart der entsprechenden Komponente der Formel III mit Chlorwasserstoff bzw. Bromwasserstoff umgesetzt, zweckmässigerweise in t-Butanol, wobei es vorteilhaft sein kann, eine geringe Menge Molekularsieb beizufügen; der als Komponente der Formel IV eingesetzte Chlorwasserstoff bzw. Bromwasserstoff dient dabei gleichzeitig zur Herbeiführung der notwendigen sauren Bedingungen. Unter Umständen braucht die Komponente der Formel III nicht als solche eingesetzt zu werden: sollen beispielsweise im Endprodukt der Formel I $R^9$ und $R^{10}$ je Wasserstoff, $R^{11}$ und $R^{12}$ je Methyl und X Chlor oder Brom bedeuten, so kann man eine entsprechende Verbindung der Formel II in t-Butanol mit Chlorwasserstoff bzw. Bromwasserstoff zur Reaktion bringen; dabei wird die entsprechende Komponente

der Formel III, d.h. Isobutylen, aus dem t-Butanol unter Einwirkung des Chlorwasserstoffes bzw. Bromwasserstoffes in situ gebildet. Die Reaktion erfolgt zweckmässigerweise bei etwa Raumtemperatur und dauert, je nach den übrigen Reaktionsparametern, etwa eine halbe Stunden bis etwa vier Tage.

Verwendet man als Komponente der Formel IV Wasser, so erhält man eine entsprechende Verbindung der Formel I, worin X Hydroxy bedeutet. Gemäss dieser Ausführungsform des vorliegenden Verfahrensaspekts werden die Komponenten der Formeln II und III unter sauren wässerigen Bedingungen miteinander umgesetzt, beispielsweise in wässeriger Salzsäure, wässeriger Methansulfonsäure oder dgl. wobei es unter Umständen zweckmässig sein kann, ein unter den Reaktionsbedingungen inertes, mit Wasser mischbares organisches Lösungsmittel beizufügen, wie etwa Tetrahydrofuran o. dgl.

Verwendet man als Komponente der Formel IV einen Alkohol der obigen Formel VI, so erhält man eine entsprechende Verbindung der Formel I, worin X einen Rest der Formel $-OR^{17}$ und $R^{17}$ einen Rest der obigen Formel (k) bedeuten. Der als Komponente der Formel IV eingesetzte Alkohol der Formel VI kann zweckmässigerweise gleichzeitig auch als Lösungsmittel dienen. Als Säure verwendet man zweckmässigerweise Methansulfonsäure, Hexafluorphosphorsäure, Tetrafluorborsäure oder dgl. Die Reaktion erfolgt zweckmässigerweise bei Raumtemperatur, und dauert, je nach den übrigen Reaktionsparametern, etwa 20 Minuten bis etwa 20 Stunden.

Aspekt b) des erfindungsgemässen Verfahrens liefert Verbindungen der Formel I, worin entweder $R^9$ und $R^{10}$ je Wasserstoff oder niederes Alkyl und $R^{11}$ und $R^{12}$ je niederes Alkyl bedeuten oder zwei der Substituenten $R^9$ und $R^{10}$ oder $R^9$ und $R^{11}$ oder $R^{11}$ und $R^{12}$ zusammen mit dem oder den Kohlenstoffatom(en), an welche(s) sie gebunden sind, einen 5-, 6- oder 7-gliedrigen carbocyclischen Ring und die beiden übrigen der Substituenten $R^9$, $R^{10}$, $R^{11}$ und $R^{12}$ je Wasserstoff oder niederes Alkyl bedeuten und worin X einen Rest der Formel $-OR^{17}$ und $R^{17}$ einen Rest der Formel (k) bedeuten. Als Komponente der Formel V verwendet man Wasser oder einen Alkohol der Formel VI. Die Reaktion erfolgt zweckmässigerweise unter sauren Bedingungen, d.h. das Ausgangsprodukt der Formel I, worin X Chlor oder Brom bedeutet, wird mit wässeriger Säure (z.B. verdünnter Salzsäure) bzw. mit einer Lösung einer Säure, wie Methansulfonsäure oder dgl., im entsprechenden Alkohol der Formel VI umgesetzt. Unter Umständen kann es von Vorteil sein, ein mit Wasser bzw. dem Alkohol der Formel Vi mischbares, unter den Reaktionsbedingungen inertes organisches Lösungsmittel beizufügen. Die Reaktion erfolgt zweckmässigerweise bei etwa Raumtemperatur und dauert, je nach den übrigen Reaktionsparametern, einige (z.B. etwa 5 bis 15) Stunden.

Aspekt c) des erfindungsgemässen Verfahrens liefert Verbindungen der Formel I, worin $R^9$ zusammen mit X eine zusätzliche Kohlenstoff-Kohlenstoff-Bindung, $R^{10}$ Wasserstoff oder niederes Alkyl und $R^{11}$ und $R^{12}$ je niederes Alkyl bedeuten. Die Dehydratisierung gemäss diesem Verfahrensaspekt erfolgt nach allgemein üblichen und jedem Fachmann geläufigen Methoden. Als Dehydratisierungsmittel verwendet man Polyphosphorsäureäthylester, Polyphosphorsäure, oder dgl. Zweckmässigerweise arbeitet man in einem unter den Reaktionsbedinungen inerten organischen Medium, beispielsweise in einem halogenierten Kohlenwasserstoff, wie Chloroform, 1,2-Dichloräthan, einem aromatischen Kohlenwasserstoff, wie Toluol, Benzol, oder dgl. oder in einem Gemisch zweier oder mehrerer derartiger Lösungsmittel, wie Chloroform/Toluol. Die Reaktion erfolgt zweckmässigerweise bei Rückflusstemperatur und dauert, je nach den übrigen Reaktionsparametern, meherere (z.B. etwa 2 bis 3) Tage.

Aspekt d) des erfindungsgemässen Verfahrens liefert Verbindungen der Formel I, worin $R^9$ und $R^{10}$ je Wasserstoff oder niederes Alkyl, $R^{11}$ und $R^{12}$ je niederes Alkyl und X einen Rest der Formel $-OR^{17}$ bedeuten, wobei $R^{17}$ Acyl oder einen Rest der Formel (k) bedeutet, worin $R^{19}$ Acyloxy-niederes-Alkyl oder Acyloxy-niederes-Alkoxy-niederes Alkyl bedeutet. Die Acylierung erfolgt nach allgemein üblichen und jedem Fachmann geläufigen Methoden, zweckmässigerweise mittels eines reaktionsfähigen Derivats der dem einzuführenden Acylrest entsprechenden Säure, beispielsweise also mittels eines Säureanhydrids, eines Säurehalogenids usw. Die Einführung einer Acetylgruppe kann z.B. zweckmässigerweise dadurch erfolgen, dass man dass Ausgangsprodukt der Formel I in Essigsäure mit Acetanhydrid umsetzt, wobei es unter Umständen von Vorteil sein kann, eine geringe Menge von Perchlorsäure, Toluolsulfonsäure oder dgl. beizufügen. Die Acylierung erfolgt zweckmässigerweise bei etwa Raumtemperatur und dauert, je nach den übrigen Reaktionsparametern, einige (z.B 4 bis 6) Stunden.

Je nach der Natur der Ausgangsprodukte und den verwendeten Reaktionsbedingungen können die erhaltenen Produkte als Salze oder als innere Salze isoliert werden. Erwünschtenfalls können innere Salze in pharmazeutisch annehmbare Salze übergeführt werden, beispielsweise mit Chlorwasserstoff, Bromwasserstoff, Phosphorsäure, Schwefelsäure, Zitronensäure, Methansulfonsäure, p-Toluolsulfonsäure und dergleichen.

Die Ausgangsprodukte der Formel II sind bekannt oder nach an sich bekannten und jedem Fachmann geläufigen Methoden leicht herstellbar; zudem enthalten einige der nachfolgenden Beispiele detaillierte Angaben betreffend die Herstellung bestimmter Verbindungen der Formel II.

Wie eingangs erwähnt, besitzen die Benzimidazol-2-yl-pyridiniumverbindungen der allgemeinen Formel I wertvolle pharmakodynamische Eigenschaften.

Repräsentative Verbindungen der Formel I wurden auf ihre Antiulcuswirkung, auf ihre magensäuresekretionshemmende Wirkung sowie auf ihre Toxizität untersucht.

Zur Bestimmung der Antiulcuswirkung wurde die nachstehend beschriebene Versuchsanordnung verwendet:

Für jede Dosis einer Testsubstanz verwendet man Gruppen von je 8 männlichen Ratten mit einem Körpergewicht von 130-150 g. Vor Beginn des Versuchs erhalten die Tiere während 24 Stunden keine Nahrung, jedoch Wasser ad libitum. Verschiedene Dosen der zu prüfenden Substanzen (suspendiert in 0,5% Tragacanth) oder das Vehikel allein (Kontrolle) werden zweimal peroral verabreicht, und zwar 1 Stunde bevor und 2 Stunden nach peroraler Verabreichung von 20 mg/kg Indomethacin. Bei Kontrolltieren führt diese Dosis von Indomethacin innerhalb von 5 Stunden zu Läsionen des Magens. 6 Stunden nach der ersten Verabreichung der zu untersuchenden Substanz (bzw. des Vehikels allein) werden die Tiere getötet. Die Ratten, welche vor dem Auftreten makroskopisch sichtbarer Läsionen der Magenschleimhaut geschützt geblieben sind, werden gezählt. Als $ED_{50}$ bezeichnet man diejenige Dosis einer Testsubstanz, bei welcher 50% der Tiere vor dem Auftreten solcher Läsionen geschützt sind.

Zur Bestimmung der magensäuresekretionhemmenden Wirkung wurde die nachstehend beschriebene Versuchsanordnung verwendet:

Weiblichen und männlichen Beagle-Hunden wird ein Teil des Magenfundus vom restlichen Magen in Form einer Tasche vom Heidenhain-Typ abgetrennt (Modifikation der von Rudick et al. in J. Surgical Research 7, 383-398 (1967) beschriebenen Methode). In die Tasche wird eine Stahlkanüle eingenäht, welche durch die Bauchdecke nach aussen geleitet wird. Vor jedem Versuch erhalten die Tiere während 18 Stunden keine Nahrung, jedoch Wasser ad libitum. Während des Versuchs sind sie wach und stehen, und ihre Magensäuresekretion wird durch intravenöse Infusion von 4-Methylhistamin, einem selektiven Agonisten der Histamin-$H_2$-Rezeptoren, stimuliert. Die Magensäureproduktion wird in 15-Minuten-Fraktionen des Magentaschensaftes bestimmt. Sobald die Magensäureproduktion einen konstanten Wert aufweist, werden die zu prüfenden Substanzen, als trockene Pulver in Gelatinekapseln gefüllt, oral verabreicht. Als $ED_{50}$ bezeichnet man diejenige Dosis einer Testsubstanz, welche bei den behandelten Tieren im Vergleich zu Kontrolltieren eine 50%ige Hemmung der durch 4-Methylhistamin hervorgerufenen Magensäureproduktion bewirkt.

In der nachfolgenden Tabelle werden für eine Reihe repräsentativer Verbindungen der Formel I die Resultate der Prüfung auf ihre Antiulcuswirkung und auf ihre magensekretionshemmende Wirkung wiedergegeben. Ausserdem enthält diese Tabelle Angaben über die akute Toxizität ($DL_{50}$ bei einmaliger oraler Verabreichung an Mäuse).

| Verbindung | Anti-Ulcus, $ED_{50}$ mg/kg p.o. | Magensäuresekretionshemmung, $ED_{50}$ mg/kg p.o. | Toxizität, DL 50 mg/kg p.o. |
|---|---|---|---|
| A | 4.4 | 4.9 | 2500-5000 |
| B | 6.1 | 3.9 | 2500-5000 |
| C | 6.0 | 5.5 | 1000-2000 |
| D | 7.0 | 4.8 | 2500-5000 |
| E | 3.2 | 3.0 | 2500-5000 |
| F | 1.9 | 1.8 | 1250-2500 |
| G | 2.8 | 1.5 | 2500-5000 |
| H | 3.2 | 4.3 | - |
| I | 4.1 | 3.8 | 2500-5000 |
| J | 4.6 | 3.7 | >5000 |

A = 2-[[[2-(2-Hydroxyäthoxy)-2-methylpropyl]thio]methyl]-4-methoxy-3-methyl-1-[5-(trifluormethyl)-2-benzimidazolyl]pyridinium-methansulfonat.

B = Intramolekular deprotonisiertes 2-[[[2-(2-Hydroxyäthoxy)-2-methylpropyl]thio]methyl]-4-methoxy-3-methyl-1-[5-(trifluormethyl)-2-benzimidazolyl]pyridiniumkation;

C = 4-Methoxy-2-[[[2-(2-methoxyäthoxy)-2-methylpropyl]thio]methyl]-3-methyl-1-[5-(trifluormethyl)-2-benzimidazolyl]pyridinium-methansulfonat;

D = Intramolekular deprotonisiertes 4-Methoxy-2-[[[2-(2-methoxyäthoxy)-2-methylpropyl]thio]methyl]-3-methyl-1-[5-(trifluormethyl)-2-benzimidazolyl]pyridiniumkation;

E = 2-[[(2-Acetoxy-2-methylpropyl)thio]methyl]-4-methoxy-3-methyl-1-[5-(trifluormethyl)-2-benzimidazolyl]pyridinium-methansulfonat;

F = 2-[[(2-Acetoxy-2-methylpropyl)thio]methyl]-4-methoxy-3-methyl-1-[5-(trifluormethyl)-2-benzimidazolyl]pyridinium-chlorid;

G = Intramolekular deprotonisiertes 2-[[(2-Acetoxy-2-methylpropyl)thio]methyl]-4-methoxy-3-methyl-1-[5-(trifluormethyl)-2-benzimidazolyl]pyridiniumkation;

H = 2-[[(2-Aethoxy-2-methylpropyl)thio]methyl]-4-methoxy-3-methyl-1-[5-(trifluormethyl)-2-benzimidazolyl]pyridinium-methansulfonat;

I = 2-[[(2-Aethoxy-2-methylpropyl)thio]methyl]-4-methoxy-3-methyl-1-[5-(trifluormethyl)-2-benzimidazolyl]pyridinium-chlorid; und

J = Intramolekular deprotonisiertes 2-[[(2-Aethoxy-2-methylpropyl)thio]methyl]-4-methoxy-3-methyl-1-[5-(trifluormethyl)-2-benzimidazolyl]pyridiniumkation.

Die eingangs definierten Verbindungen können als Heilmittel, z.B. in Form pharmazeutischer Präparate Verwendung finden. In erster Linie kommt orale Verabreichung in Form von festen pharmazeutischen Präparaten, wie Tabletten, Lacktabletten, Dragées, Hartgelatinekapseln und Weichgelatinekapseln in Frage. Orale Verabreichung in Form flüssiger pharmazeutischer Präparate, wie Lösungen, Emulsionen und Suspensionen, rektale Verabreichung, z.B. in Form von Suppositorien, oder parenterale Verabreichung, z.B. in Form von Injektionslösungen, sind zwar weniger in Betracht zu ziehen aber auch nicht auszuschliessen.

Arzneimittel, enthaltend eine der eingangs definierten Verbindungen sind ebenfalls Gegenstand der vorliegenden Erfindung. Die Herstellung derartiger Arzneimittel kann dadurch erfolgen, dass man eine oder mehrere der eingangs definierten Verbindungen und erwünschtenfalls einen oder mehrere andere therapeutische Wirkstoffe zusammen mit einem oder mehreren therapeutisch inerten Excipientien in eine galenische Darreichungsform bringt.

Zur Herstellung von Tabletten, Lacktabletten, Dragées und Hartgelatinekapseln können die eingangs definierten Verbindungen mit pharmazeutisch inerten, anorganischen oder organischen Excipientien verarbeitet werden. Als solche Excipientien kann man z.B. für Tabletten, Dragées und Hartgelatinekapseln Lactose, Maisstärke oder Derivate davon, Talk, Stearinsäure oder deren Salze etc. verwenden. Zur Herstellung von magensaftresistenten pharmazeutischen Präparaten ist noch ein magensaftresistenter Lack

aufzubringen, welcher z.B. aus Hydroxypropylmethylcellulosephthalat bestehen kann.

Für Weichgelatinekapseln eignen sich als Excipientien z.B. vegetabile Oele, Wachse, Fette, halbfeste und flüssige Polyole etc.

Zur Herstellung von Lösungen und Sirupen eignen sich als Excipientien z.B. Wasser, Polyole, Saccharose, Invertzucker, Glucose und dgl.

Für Suppositorien eignen sich als Excipientien z.B. natürliche oder gehärtete Oele, Wachse, Fette, halbflüssige oder flüssige Polyole und dgl.

Für Injektionslösungen eignen sich als Excipientien z.B. Wasser, Alkohole, Polyole, Glycerin, vegetabile Oele etc.

Die pharmazeutischen Präparate können daneben noch Konservierungsmittel, Lösevermittler, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel oder Antioxidantien enthalten. Sie können auch noch andere therapeutisch wertvolle Stoffe enthalten.

Erfindungsgemäss kann man die eingangs definierten Verbindungen bei der Bekämpfung bzw. Verhütung von Krankheiten verwenden, beispielsweise bei der Bekämpfung bzw. Verhütung von Ulcus ventriculi und duodeni. Die Dosierung kann innerhalb weiter Grenzen variieren und ist natürlich in jedem einzelnen Fall den individuellen Gegebenheiten anzupassen. Im allgemeinen dürfte bei oraler Verabreichung eine Tagesdosis von etwa 30-400 mg und bei intravenöser Verabreichung eine Tagesdosis von etwa 30-400 mg angemessen sein.

Gegenstand der Erfindung ist auch die Verwendung der eingangs definierten Verbindungen zur Herstellung von Arzneimitteln für die Bekämpfung bzw. Verhütung von Ulcus ventriculi und duodeni.

In den nachfolgenden Beispielen, welche die vorliegende Erfindung illustrieren, ihren Umfang aber in keiner Weise beschränken sollen, sind sämtliche Temperaturen in Celsiusgraden angegeben.

## Beispiel 1

Eine Suspension von 15 g 2-[[(4-Methoxy-3-methyl-2-pyridyl)methyl]sulfinyl]-5-(trifluormethyl)-benzimidazol in einer Lösung von 7,2 g Isobutylen in 130 ml tert.-Butanol wird mit 20 g Molekularsieb (Union Carbide Type 3A) und mit einer Lösung von 19,5 g gasförmigen Chlorwasserstoff in 150 ml tert.-Butanol versetzt. Das Reaktionsgemisch wird bei Raumtemperatur während 50 Minuten gerührt und anschliessend auf ein Gemisch von Eis und 1,2 l wässeriger Natriumbicarbonatlösung gegossen, worauf man Methylenchlorid zugibt. Der unlösliche Teil des Gemisches wird über Kieselgel abfiltriert, und das Filtrat wird mehrmals mit Methylenchlorid extrahiert. Die organischen Phasen werden vereinigt, mit Natriumsulfat getrocknet, filtriert und eingeengt. Der Rückstand wird in 800 ml Methylenchlorid gelöst, worauf man 28 g Kiselgel (Korngrösse: 0,04-0,06 mm) zugibt und während einer Stunde bei Raumtemperatur rührt. Man filtriert das Kieselgel ab, engt das Filtrat ein und kristallisiert den Rückstand aus Aether. Das erhaltene intramolekular deprotonisierte 2-[[(2-Chlor-2-methylpropyl)thio]methyl]-4-methoxy-3-methyl-1-[5-(trifluormethyl)-2-benzimidazolyl]pyridiniumkation zeigt einen Schmelzpunkt von 132-134° (Zersetzung).

## Beispiel 2

100 mg intramolekular deprotonisiertes 2-[[(2-Chlo-2-methylpropyl)thio]methyl]-4-methoxy-3-methyl-1-[5-(trifluormethyl)-2-benzimidazolyl]pyridiniumkation werden in 5 ml Essigester gelöst, worauf man 0,5 ml 4,7N methanolische Salzsäure zugibt, die Lösung einengt und den Rückstand aus tert.-Butyl-methyläther/Aether kristallisiert. Das erhaltene 2-[[(2-Chlor-2-methylpropyl)thio]methyl]-4-methoxy-3-methyl-1-[5-(trifluormethyl)-2-benzimidazolyl]pyridiniumchlorid zeigt einen Schmelzpunkt von 140-142° (Zersetzung).

## Beispiel 3

Eine Suspension von 5 g intramolekular deprotensiertem 2-[[(2-Chlor-2-methylpropyl)thio]methyl]-4-methoxy-3-methyl-1-[5-(trifluormethyl)-2-benzimidazolyl]pyridiniumkation in 100 ml 1N wässeriger Salzsäure

wird neun Stunden bei Raumtemperatur gerührt und dann auf ein Gemisch von Eis und wässeriger Natriumbicarbonatlösung gegossen, worauf man mehrmals mit Methylenchlorid extrahiert. Die vereinigten Methylenchloridextrakte werden mit Natriumsulfat getrocknet, filtriert und eingeengt. Der Rückstand wird zweimal aus Aether umkristallisiert, wobei man intramolekular deprotonisiertes 2-[[(2-Hydroxy-2-methylpropyl)thio]methyl]-4-methoxy-3-methyl-1-[5-(trifluormethyl)-2-benzimidazolyl]pyridiniumkation vom Schmelzpunkt 144-145° erhält.

Beispiel 4

Eine Lösung von 200 mg Methansulfonsäure und etwa 1 g gasformigem Isobutylen in 20 ml Methanol wird mit 370 mg 2-[[(4-Methoxy-3-methyl-2-pyridyl)methyl]sulfinyl]-5-(trifluormethyl)benzimidazol versetzt, worauf man bei Raumtemperatur während 18 Stunden rührt und dann das Reaktionsgemisch eindampft. Man versetzt den Rückstand mit Methylenchlorid und wässeriger Natriumbicarbonatlösung, trennt die Methylenchloridphase ab und extrahiert die wässerige Phase mit Methylenchlorid. Die vereinigten organischen Phasen werden getrocknet und eingeengt. Durch Umkristallisation des Rückstandes aus Aether/n-Hexan erhält man intramolekular deprotonisiertes 4-Methoxy-2-[[(2-methoxy-2-methylpropyl)thio]methyl]-3-methyl-1-[5-(trifluormethyl)-2-benzimidazolyl]pyridiniumkation vom Schmelzpunkt 155-156°.

Beispiel 5

Eine Lösung von 200 mg Methansulfonsäure und etwa 0,5 g gasförmigem Isobutylen in 20 ml Aethanol wird mit 350 mg 2-[[(4-Methoxy-3-methyl-2-pyridyl)methyl]sulfinyl]-5-(trifluormethyl)benzimidazol versetzt, worauf man bei Raumtemperatur während 18 Stunden rührt, das Reaktionsgemisch einengt und den Rückstand mit Methylenchlorid und wässeriger Natriumbicarbonatlösung versetzt. Hierauf trennt man die Methylenchloridphase ab und extrahiert die wässerige Phase mit Methylenchlorid. Die vereinigten organischen Phasen werden getrocknet und eingeengt. Durch Umkristallisation des Rückstands aus Aether erhält man intramolekular deprotonisiertes 2-[[(2-Aethoxy-2-methylpropyl)thio]methyl]-4-methoxy-3-methyl-1-[5-(trifluormethyl)-2-benzimidazolyl]pyridiniumkation vom Schmelzpunkt 132-134°.

Beispiel 6

10 ml mit Isobutylen gesättigter Aethylenglykolmonomethyläther wird mit 192 mg Methansulfonsäure und 370 mg 2-[[(4-Methoxy-3-methyl-2-pyridiyl)methyl]sulfinyl]-5-(trifluormethyl)benzimidazol versetzt. Die Lösung wird unter Isobutylenatmosphäre während vier Stunden bei Raumtemperatur gerührt und dann eingeengt. Der Rückstand wird mit Methylenchlorid und wässeriger Natriumbicarbonatlösung versetzt, worauf man die Methylenchloridphase abtrennt und die wässerige Phase mit Methylenchlorid extrahiert. Die vereinigten organischen Phasen werden getrocknet und eingeengt. Der Rückstand wir aus Aether kristallisiert, und man erhält intramolekular deprotonisiertes 4-Methoxy-2-[[[2-(2-methoxyäthoxy)-2-methylpropyl]-thio]methyl]-3-methyl-1-[5-(trifluormethyl)-2-benzimidazolyl]pyridiniumkation vom Schmelzpunkt 110-112° (Zersetzung).

Beispiel 7

4 ml mit Isobutylen gesättigtes Aethylenglykol wird mit 200 mg Methansulfonsäue und 370 mg 2-[[(4-Methoxy-3-methyl-2-pyridyl)methyl]sulfinyl]-5-(trifluormethyl)benzimidazol versetzt. Die Lösung wird unter Isobutylenatmosphäre während neun Stunden bei Raumtemperatur gerührt und dann auf Eis und Natriumbicarbonat gegossen. Die entstandene wässerige Lösung wird mehrmals mit Methylenchlorid extrahiert. Die vereinigten organischen Phasen werden getrocknet und eingedampft. Der Rückstand wird an Kieselgel mit

Methylenchlorid/Methanol (10:1) als Elutionsmittel chromatographiert, wobei die Methode der Mitteldruck-Flashchromatographie verwendet wird. Durch Umkristallisation aus Aether/n-Hexan erhält man intramolekular deprotonisieres 2-[[[2-(2-Hydroxyäthoxy)-2-methylpropyl]thio]methyl]-4-methoxy-3-methyl-1-[5-(trifluormethyl)-2-benzimidazolyl]pyridiniumkation vom Schmelzpunkt 60-70°.

Beispiel 8

a) 10,6 g 5-Fluor-2-benzimidazolthiol werden in 570 ml Alkohol suspendiert und mit 13,1 g 2-Chlormethyl-4-methoxy-3-methylpyridin-hydrochlorid versetzt. Unter Eiskühlung tropft man eine Lösung von 5 g Natriumhydroxid in 130 ml Wasser zu, lässt das Gemisch über Nacht am Rückfluss kochen und engt es dann im Vakuum auf ca. 1/3 des Volumens ein. Nach Zugabe von 500 ml Wasser werden die entstandenen Kristalle abfiltriert und zuerst mit Wasser und dann mit Aether gründlich gewaschen. Man erhält 5-Fluor-2-[[(4-methoxy-3-methyl-2-pyridyl)methyl]thio]benzimidazol vom Schmelzpunkt 128°.

b) 2,0 g 5-Fluor-2-[[(4-methoxy-3-methyl-2-pyridyl)methyl]thio]benzimidazol, gelöst in 100 ml Methylenchlorid, werden mit 1,2 g Kaliumcarbonat versetzt. Bei -30° gibt man 1,6 g m-Chlorperbenzoesäure zu, rührt die Lösung 5 Minuten weiter und giesst sie anschliessend in ein Gemisch von 30 ml gesättigter Natriumbicarbonatlösung und 30 ml Wasser. Die organische Phase wird abgetrennt, über Natriumsulfat getrocknet, mit 5,0 ml Triäthylamin versetzt und im Vakuum eingedampft. Kristallisation des Rückstandes aus Methylenchlorid/Petroläther (tiefs.)/Aether ergibt 5-Fluor-2-[[(4-methoxy-3-methyl-2-pyridyl)methyl]-sulfinyl]benzimidazol vom Schmelzpunkt 175°.

c) Eine Lösung von 3,2 g 5-Fluor-2-[[(4-methoxy-3-methyl-2-pyridyl)methyl]sulfinyl]benzimidazol in 200 ml tert. Butanol wird 30 Minuten mit Isobutylengas gesättigt, dann mit einer frisch zubereiteten Lösung von 22,4 g Chlorwasserstoff-Gas in 200 ml tert. Butanol versetzt, über Nacht bei Raumtemperatur gerührt und anschliessend im Vakuum eingdampft. Der Rückstand wird in Methylenchlorid aufgenommen, worauf die Lösung mit 100 ml gesättigter Natriumbicarbonatlösung extrahiert, mit Wasser neutralgewaschen, getrocknet und im Vakuum eingeengt wird. Kristallisation des Rückstandes aus Methylenchlorid/Petroläther (tiefs.) ergibt intramolekular deprotonisiertes 2-[[(2-Chlor-2-methylpropyl)thio]methyl]-1-(5-fluor-2-benzimidazolyl)-4-methoxy-3-methylpyridiniumkation vom Schmelzpunkt 136-138°.

Beispiel 9

Eine Lösung von 2 g Methansulfonsäure in 200 ml Methanol wird 30 Minuten mit Isobutylengas gesättigt, dann mit 3,2 g 5-Fluor-2-[[(4-methoxy-3-methyl-2-pyridyl)methyl]sulfinyl]benzimidazol versetzt, über Nacht bei Raumtemperatur gerührt und anschliessend im Vakuum eingedampft. Der Rückstand wird in Methylenchlorid aufgenommen, worauf die Lösung mit 100 ml gesättigter Natriumbicarbonatlösung extrahiert, mit Wasser neutralgewaschen, getrocknet und im Vakuum eingeengt wird. Kristallisation des Rückstands aus Methylenchlorid/Petroläther (tiefs.) ergibt intramolekular deprotonisiertes 1-(5-Fluor-2-benzimidazolyl)-4-methoxy-2-[[(2-methoxy-2-methylpropyl)thio]methyl]-3-methylpyridiniumkation vom Schmelzpunkt 142-143°.

Beispiel 10

450 mg intramolekular deprotonisiertes 2-[[(2-Aethoxy-2-methylpropyl)thio]methyl]-4-methoxy-3-methyl-1-[5-(trifluormethyl)-2-benzimidazolyl]pyridiniumkation werden in Methylenchlorid gelöst, worauf man 2 ml 4,7N methanolische Salzsäure zugibt, die Lösung einengt und den Rückstand aus Aether kristallisiert. Das erhaltene 2-[[(2-Aethoxy-2-methylpropyl)thio]methyl]-4-methoxy-3-methyl-1-[5-(trifluormethyl)-2-benzimidazolyl]pyridiniumchlorid zeigt einen Schmelzpunkt von 119-120°.

Beispiel

370 mg intramolekular deprotonisiertes 2-[[[2-(2-Hydroxyäthoxy)-2-methylpropyl]thio]methyl]-4-methoxy-3-methyl-1-[5-(trifluormethyl)-2-benzimidazolyl]pyridiniumkation werden in Methanol gelöst, worauf man 100 mg Methansulfonsäure zugibt, die Lösung einengt und den Rückstand aus Aether/Essigester kristallisiert. Das erhaltene 2-[[[2-(2-Hydroxyäthoxy)-2-methylpropyl]thio]methyl]-4-methoxy-3-methyl-1-[5-(trifluormethyl)-2-benzimidazolyl]pyridinium-methansulfonat zeigt einen Schmelzpunkt von 105-107° (Zersetzung).

Beispiel 12

483,5 mg intramolekular deprotonisiertes 4-Methoxy-2-[[[2-(2-methoxyäthoxy)-2-methylpropyl]thio]-methyl]-3-methyl-1-[5-trifluormethyl)-2-benzimidazolyl)pyridiniumkation werden in 5 ml Methanol gelöst, worauf man 96 mg Methansulfonsäure zugibt, die Lösung einengt und den Rückstand mehrmals in Essigester löst und die Lösung jeweils einengt. Der harzartige Rückstand wird am Hochvakuum getrocknet, wobei man 4-Methoxy-2-[[[2-(2-methoxyäthoxy)-2-methylpropyl]thio]methyl]-3-methyl-1-[5-(trifluormethyl)-2-benzimidazolyl]pyridinium-methansulfonat als Schaum erhält. Die Mikroanalyse zeigt folgende Werte:

Summenformel; $C_{23}H_{29}F_3N_3O_3S$ 1:1$CH_3SO_3$; MG 579,65

Ber.:

C 49,73 % H 5,56 % N 7,25 % S 11,06 %

Gef.:

C 49,59 % H 5,76 % N 7,19 % S 10,97 %

Beispiel 13

Eine Lösung von 160 mg gasförmigem Isobutylen und 350 mg 2-[[(4-Methoxy-3-methyl-2-pyridyl)-methyl]sulfinyl]-5-(trifluor methyl)benzimidazol in 5 ml Diäthylenglykolmonomethyläther wird mit 200 mg Methansulfonsäure versetzt, worauf man bei Raumtemperatur während 18 Stunden rührt und dann das Reaktionsgemisch mit 100 ml gesättigter wässeriger Natriumbicarbonatlösung neutralisiert. Die Natriumbicarbonatlösung wid mehrmals mit Aether extrahiert; die vereinigten organischen Phasen werden übr Natriumsulfat getrocknet und eingeengt. Der Rückstand wird an Kieselgel mit Methylenchlorid/Methanol (20:1) als Elutionsmittel chromatographiert, wobei die Methode der Mitteldruck-Flashchromatographie verwendet wird. Durch Umkristallisation aus n-Hexan erhält man intramolekular deprotonisiertes 4-Methoxy-2-[[-[2-[2-(2-methoxyäthoxy)äthoxy]-2-methylpropyl]thio]methyl]-3-methyl-1-[5-(trifluormethyl)-2-benzimidazolyl]-pyridiniumkation vom Schmelzpunkt 103-106°.

Beispiel 14

Eine Lösung von 500 mg intramolekular deprotonisiertem 2-[[(2-Hydroxy-2-methylpropyl)thio]methyl]-4-methoxy-3-methyl-1-[5-(trifluormethyl)-2-benzimidazolyl]pyridiniumkation in 1,5 ml Essigsäure und 3 ml Acetanhydrid wir mit 3 Tropfen Perchlorsäure versetzt und während 5 Stunden bei Raumtemperatur gerührt. Man neutralisiert das Reaktionsgemisch mit gesättigter wässeriger Natriumbicarbonatlösung, extrahiert mehrmals mit Methylenchlorid, trocknet die Extrakte über Natriumsulfat, filtriert und dampft das Methylenchlorid ab. Der Rückstand wird an Kieselgel mit Methylenchlorid/Methanol (10:1) als Elutionsmittel chromatographiert, wobei die Methode der Mitteldruck-Flashchromatographie verwendet wird. Durch Kristallisation aus Aether/n-Hexan erhält man intramolekular deprotonisiertes 2-[[(2-Acetoxy-2-methylpropyl)thio]-methyl]-4-methoxy-3-methyl-1-[5-(trifluormethyl)-2-benzimidazolyl] pyridiniumkation vom Schmelzpunkt 71-78°.

## Beispiel 15

467,5 mg intramolekular deprotonisiertes 2-[[2-Acetoxy-2-methylpropyl)thio]methyl]-4-methoxy-3-methyl-1-[5-(trifluormethyl)-2-benzimidazolyl]pyridiniumkation werden in 5 ml Methanol gelöst, worauf man 96 mg Methansulfonsäure zugibt, die Lösung einengt, den Rückstand mehrmals in Essigester löst und die Lösung jeweils wieder einengt. Der harzartige Rückstand wird am Hochvakuum getrocknet, wobei 2-[[(2-Acetoxy-2-methylpropyl)thio]methyl]-4-methoxy-3-methyl-1-[5-(trifluormethyl)-2-benzimidazolyl]pyridinium-methansulfonat (1:1) als Schaum entsteht. Die Mikroanalyse zeigt folgende Werte:
Summenformel; $C_{22}H_{25}F_3N_3O_3S$ 1:1 $CH_3SO_3$ ; MG 563,61
Ber.:
C 49,02 % H 5,01 % N 7,46 % S 11,38 %
Gef.:
C 48,87 % H 5,28 % N 7.30 % S 11,00 %

## Beispiel 16

Eine Lösung von 1 g Methansulfonsäure und etwa 1 g gasförmigem Isobutylen in 15 ml n-Propanol wird mit 2 g 2-[[(4-Methoxy-3-methyl-2-pyridyl)methyl]sulfinyl]-5-(trifluormethyl)benzimidazol versetzt, worauf man das Gemisch bei Raumtemperatur während 18 Stunden in einem mit einer Kühlfalle (Aceton/Trockeneis) versehenen Kolben rührt und dann eindampft. Man versetzt den Rückstand mit Methylenchlorid und wässeriger Natriumbicarbonatlösung, trennt die Methylenchloridphase ab und extrahiert die wässerige Phase mit Methylenchlorid. Die vereinigten organischen Phasen werden getrocknet und eingeengt. Der Rückstand wird an Kieselgel mit Methylenchlorid/Methanol (20:1) als Elutionsmittel chromatographiert, wobei die Methode der Mitteldruck-Flashchromatographie verwendet wird. Durch Kristallisation aus Aether/n-Hexan erhält man intamolekular deprotnisiertes 4-Methoxy-3-methyl-2-[[(2-methyl-2-propoxypropyl)thio]methyl]-1-[(5-(trifluormethyl)-2-benzimidazolyl]pyridiniumkation vom Schmelzpunkt 106-108°.

## Beispiel 17

Eine Lösung von 2 g Methansulfonsäure und etwa 2 g gasförmigen Isobutylen in 5 ml Diäthylenglykol wird mit 3 g 2-[[(4-Methoxy-3-methyl-2-pyrdidyl)methyl]sulfinyl]-5-(trifluormethyl)benzimidazol versetzt, worauf man das Gemisch bei Raumtemperatur während 18 Stunden in einem mit einer Kühlfalle (Aceton/Trockeneis) versehenen Kolben rührt und dann eindampft. Man versetzt den Rückstand mit Methylenchlorid und wässeriger Natriumbicarbonatlösung, trennt die Methylenchloridphase ab und extrahiert die wässerige Phase mit Methylenchlorid. Die vereinigten organischen Phasen werden getrocknet und eingeengt. Der Rückstand wird an Kieselgel (Korngrösse: 0,04-0,06 mm) mit Methylenchlorid/Methanol (20:1) als Elutionsmittel chromatographiert, wobei die Methode der Mitteldruck-Flashchromatographie verwendet wird.

Das gereinigte intramolekular deprotonisierte 2-[[[2-[2-(2-Hydroxyäthoxy)äthoxy]-2-methylpropyl]thio]-methyl]-4-methoxy-1-[5-(trifluormethyl)-2-benzimidazolyl]pyridiniumkation wird mehrmals in Essigester gelöst, und die erhaltene Lösung wird jeweils eingeengt. Der harzartige Rückstand wird am Hochvakuum getrocknet, wobei ein Schaum entsteht. Die Mikroanalyse zeigt fogende Werte:
Summenformel; $C_{24}H_{30}F_3N_3O_4S$; MG 513, 57
Ber.:
C 56,13 % H 5,89 % N 8,18 % S 6,24 %
Gef.:
C 55,74 % H 6,06 % N 8,00 % S 6,21 %

Beispiel 18

Eine Lösung von 1 g Methansulfonsäure und etwa 1 g gasförmigen Isobutylen in 15 ml n-Butanol wird mit 2 g 2-[[(4-Methoxy-3-methyl-2-pyridyl)methyl]sulfinyl]-5-(trifluormethyl)benzimidazol versetzt worauf man das Gemisch bei Raumtemperatur während drei Stunden in einem mit einer Kühlfalle (Aceton/Trockeneis) versehenen Kolben rührt und dann eindampft. Man versetzt den Rückstand mit Methylenchlorid und wässeriger Natriumbicarbonatlösung, trennt die Methylenchloridphase ab und extrahiert die wässerige Phase mit Methylenchlorid. Die vereinigten organischen Phasen werden getrocknet und eingeengt. Der Rückstand wird an Kieselgel mit Methylenchlorid/Methanol (20:1) als Elutionsmittel chromatographiert, wobei die Methode der Mitteldruck-Flashchromatographie verwendet wird. Durch Kristallisation aus Aether/n-Hexan erhält man intramolekular deprotonisiertes 2-[[)2-Butoxy-2-methylpropyl)thio]methyl]-4-methoxy-3-methyl-1-[5-(trifluormethyl)-2-benzimidazolyl]pyridiniumkation vom Schmelzpunkt 78-80°.

Beispiel 19

Eine Lösung von 19 g Polyphosphorsäure-äthylester und 6,3 g intramolekular deprotonisiertem 2-[[(2-Hydroxy-2-methylpropyl)thio]methyl]-4-methoxy-3-methyl]-1-[5-(trifluormethyl)-2-benzimidazolyl]-pyridiniumkation in 150 ml Chloroform und 45 ml Toluol wird während etwa 60 Stunden am Rückfluss erhitzt. Man giesst das Reaktionsgemisch auf gesättigte wässerige Natriumcarbonatlösung, extrahiert mehrmals mit Methylenchlorid, trocknet die vereinigten Extrakte über Natriumsulfat und dampft das Methylenchlorid ab. Der Rückstand wird an Kieselgel mit Methylenchlorid/Methanol (20:1) als Elutionsmittel chromatographiert, wobei die Methode der Mitteldruck-Flashchromatographie verwendet wird. Durch Kristallisation aus Aether/n-Hexan erhält man intramolekular deprotonisiertes 4-Methoxy-3-methyl-2-[[(2-methylpropenyl)thio]methyl]-1-[5-(trifluormethyl)-2-benzimidazolyl]pyridiniumkation vom Schmelzpunkt 126-127°.

Beispiel 20

60 mg intramolekular deprotonisiertes 4-Methoxy-3-methyl-2-[[(2-methylpropenyl)thio]methyl]-1-[5-(trifluormethyl)-2-benzimidazolyl]pyridiniumkation werden in Methylenchlorid gelöst, worauf man 0,5 ml 4,7N methanolische Salzsäure zugibt, die Lösung einengt und den Rückstand aus Essigester/Aether kristallisiert. Das erhaltene 4-Methoxy-3-methyl-2-[[(2-methylpropenyl)thio]methyl]-1-[5-(trifluormethyl)-2-benzimidazolyl]-pyridiniumchlorid zeigt einen Schmelzpunkt von 142-144°.

Beispiel 21

Eine Lösung von 2 g Methansulfonsäure in 200 ml abs. Aethanol wird 45 Minuten mit Isobutylengas gesättigt und dann mit 3,2 g 5-Fluor-2-[[(4-methoxy-3-methyl-2-pyridyl)methyl]sulfinyl]benzimidazol versetzt, worauf man über Nacht bei Raumtemperatur rührt und anschliessend im Vakuum eindampft. Der Rückstand wird in Methylenchlorid aufgenommen, worauf die Lösung mit 100 ml gesättigter Natriumbicarbonatlösung extrahiert, neutralgewaschen, getrocknet und im Vakuum eingeengt wird. Chromatographie an Silicagel mit Methanol-Methylenchlorid (5:95) und anschliessende Kristallisation aus Methylenchlorid/ Petroläther (tiefs.) ergibt intramolekular deprotonisiertes (-)-2-[[(2-Aethoxy-2-methylpropyl)thio]methyl]-1-(5-fluor-2-benzimidazolyl)-4-methoxy-3-methylpyridiniumkation vom Schmelzpunkt 121-122°.

Beispiel 22

Eine Lösung von 2 g Methansulfonsäure in 200 ml Dioxan wird 45 Minuten mit Isobutylengas gesättigt und dann mit 3,2 g 5-Fluor-2-[[(4-methoxy-3-methyl-2-pyridyl)methyl]sulfinyl]benzimidazol versetzt. Nach 10 Minuten Rühren gibt man 5 ml Aethylenglykol zu, worauf das Gemisch 48 Stunden bei Raumtemperatur

gerührt und anschliessend im Vakuum eingdampft wird. Der Rückstand wird in Methylenchlorid aufgenommen, worauf die Lösung mit 100 ml gesättigter Natriumbicarbonatlösung extrahiert, mit Wasser neuralgewaschen, getrocknet und im Vakuum eingeengt wird. Kristallisation aus Methylenchlorid/abs. Aether/Petroläther (tiefs.) ergibt intramolekular deprotonisiertes 1-(5-Fluor-2-benzimidazolyl)-2-[[[2-(2-hydroxyäthoxy)-2-methylpropyl]thio]methyl]-4-methoxy-3-methylpyridiniumkation vom Schmelzpunkt 75-80°.

## Beispiel 23

a) 40,7 g 4,5-Difluor-o-phenylendiamin-dihydrochlorid werden in 655 ml Isopropanol suspendiert. Unter Rühren tropft man eine Lösung von 22,5 g Kaliumhydroxid in 250 ml Wasser zu und versetzt mit 39,7 g Kaliumäthylxanthogenat, worauf die Lösung über Nacht am Rückfluss gekocht, dann mit 300 ml Wasser verdünnt und mit Eisessig neutral gestellt wird. Die entstandene Suspension wird noch eine Stunde bei 60-70° gerührt. Das Isopropanol wird aus dem Gemisch im Vakuum weitgehend entfernt. Nach Zugabe von 1 l Wasser wird der Festkörper abfiltriert, mit Wasser gewaschen und dann in Aether gelöst, worauf die Lösung mit 1,2, l Wasser extrahiert, mit Aktivkohle behandelt , getrocknet und im Vakuum eingedampft wird. Der Rückstand wird in Petroläther (tiefs.) aufgeschlämmt und abfiltriert. Man erhält 5,6-Difluor-2-benzimidazolthiol vom Schmelzpunkt über 300°.

b) Eine Suspension von 23 g 5,6-Difluor-2-benzimidazolthiol in 740 ml Alkohol wird mit 22,1 g 2-Chlormethyl-4-methoxy-3-methylpyridin-hydrochlorid versetzt. Unter Eiskühlung tropft man eine Lösung von 10 g Natriumhydroxid in 350 ml Wasser zu, lässt das Gemisch über Nacht am Rückfluss kochen und engt es dann in Vakuum auf ca. 1/3 seines Volumens ein. Nach Zugabe von 1200 ml Wasser werden die Kristalle abfiltriert und hierauf zuerst mit Wasser und dann mit Aether gründlich gewaschen. Man erhält 5,6-Difluor-2-[[(4-methoxy-3-methyl-2-pyridyl)methyl]thio]benzimidazol vom Schmelzpunkt 216-218°.

c) Eine Lösung von 13,7 g 5,6-Difluor-2-[[(4-methoxy-3-methyl-2-pyridyl)methyl]thio]benzimidazol in 800 ml Methylenchlorid und 130 ml Methanol wird mit 6,9 g Kaliumcarbonat versetzt. Bei -30° gibt man unter Rühren 9,6 g m-Chlorperbenzoesäure zu, worauf die Lösung 5 Minuten weitergerührt und anschliessend in ein Gemisch von 200 ml gesättigter Natriumbicarbonatlösung und 200 ml Wasser gegossen wird. Die abgetrennte organische Phase wird über Natriumsulfat getrocknet, mit 5,0 ml Triäthylamin versetzt und im Vakuum eingedampft. Kristallisation aus Methylenchlorid-Methanol-Aether ergibt 5,6-Difluor-2-[[(4-me thoxy-3-methyl-2-pyridyl))methyl]sulfinyl]benzimidazol vom Schmelzpunkt 188-189°.

d) Eine Lösung von 2 g Methansulfonsäure in 200 ml Methanol wird 45 Minuten mit Isobutylengas gesättigt und dann mit 3,37 g 5,6-Difluor-2-[[)4-methoxy-3-methyl-2-pyridyl)methyl]sulfinyl]benzimidazol versetzt, worauf das Gemisch über Nacht bei Raumtemperatur gerührt und anschliessend im Vakuum eingedampft wird. Der Rückstand wird in Methylenchlorid aufgenommen, worauf die Lösung mit 100 ml gesättigter Natriumbicarbonatlösung extrahiert, mit Wasser neutralgewaschen, getrocknet und im Vakuum eingeengt wird. Kristallisation des Rückstandes aus Methylenchlorid/Petroläther (tiefs.) ergibt intramolekular deprotonisiertes 1-(5,6-Difluor-2-benzimidazolyl)-4-methoxy-2-[[(2-methoxy-2-methylpropyl)thio]methyl]-3-methylpyridiniumkation vom Schmelzpunkt 153-155°.

## Beispiel 24

Eine Lösung von 2 g Methansulfonsäure in 200 ml Aethanol wird 45 Minuten mit Isobutylengas gesättigt, dann mit 3,37 g 5,6-Difluor-2-[[(4-methoxy-3-methyl-2-pyridyl)methyl]sulfinyl]benzimidazol versetzt, über Nacht bei Raumtemperatur gerührt und anschliessend im Vakuum eingedampft. Der Rückstand wird in Methylenchlorid aufgenommen, worauf die Lösung mit 100 ml gesättigter Natriumbicarbonatläsung extrahiert, mit Wasser neutralgewaschen, getrocknet und im Vakuum eingeengt wird. Kristalisation des Rückstandes aus Methylenchlorid/Petroläther (tiefs.) ergibt intramolekular deprotonisiertes 1-(5,6-Difluor-2-benzimidazolyl)-4-methoxy-2-[[(2-äthoxy-2-methylpropyl)thio]methyl]-3-methylpyridiniumkation vom Schmelz- punkt 122-124°.

Beispiel 25

Eine Lösung von 3,37 g 5,6-Difluor-2-[[(4-methoxy-3-methyl-2-pyridyl)methyl[sulfinyl]benzimidazol in 200 ml tert. Butanol wird 45 Minuten mit Isobutylengas gesättigt, dann mit einer frisch zubereiteten Lösung von 23 g Chlorwasserstoff-Gas in 200 ml tert. Butanol versetzt, 48 Stunden bei Raumtemperatur gerührt und anschliessend im Vakuum eingedampft. Der Rückstand wird in Methylenchlorid aufgenommen, worauf die Lösung mit 100 ml gesättigter Natriumbicarbonatlösung extrahiert, mit Wasser neutralgewaschen, getrocknet und im Vakuum eingeengt wird. Kristallisation des Rückstandes aus Methylenchlorid/Petroläther (tiefs.) ergibt intramolekular deprotonisiertes 2-[[(2-Chlor-2-methylpropyl)thio]methyl]-1-(5,6-difluor-2-benzimidazolyl]-4-methoxy-3-methylpyridiniumkation vom Schmelzpunkt 138-140°.

Beispiel 26

500 mg 5,7-Dihydro-2[[(4-methoxy-3-methyl-2-pyridyl)methyl]sulfinyl]-5,5,7,7,-tetramethylindeno[5,6-d]-imidazol-6(1H)-on werden in 50 ml mit gasförmiger Salzsäure gesättigtem tert.-Butanol gelöst und 3 Tage bei Raumtemperatur stehengelassen. Das Reaktionsgemich wird eingeengt, und der Rückstand wird aus Essigester kristallisiert. Das erhaltene 2-[[(2-Chlor-2-methylpropyl)thio]methyl]-4-methoxy-3-methyl-1-(1,5,6,7-tetrahydro-5,5,7,7-tetramethyl-6-oxoindeno[5,6-d]imidazol-2-yl)pyridiniumchlorid zeigt einen Schmelzpunkt von 168-172° (Zersetzung).

Beispiel 27

Eine Suspension von 1,75 g 2-[[(4-Methoxy-3-methyl-2-pyridyl)methyl]sulfinyl]-5-(trifluormethyl)-benzimidazol in einer Lösung von 800 mg Isobutylen in 25 ml Triäthylenglykolmonomethyläther wird mit 1 g Methansulfonsäure versetzt und während 18 Stunden bei Raumtemperatur gerührt. Das Reaktionsgemisch wird mit gesättigter wässeriger Natriumbicarbonatlösung neutralisiert; die wässerige Phase wird mehrmals mit Aether extrahiert, und die vereinigten organischen Phasen werden über Natriumsulfat getrocknet und eingeengt. Der Rückstand wird an Kieselgel mit Aether und Methylenchlorid/Methanol(20:1) als Elutionsmittel chromatographiert, wobei die Methode der Mitteldruck-Flashchromatographie verwendet wird. Das harzartige Reaktionsprodukt wird im Hochvakuum getrocknet, wobei man intramolekular deprotonisiertes 4-Methoxy-2-[[[2-[2-(2-methoxyäthoxy)äthoxy]äthoxy]-2-methylpropyl]thio]methyl-3-methyl-1-[5-trifluormethyl)-2-benzimidazolyl]pyridiniumkation als Schaum erhalt. Die Mikroanalyse zeigt folgende Werte:

Summenformel; $C_{27}H_{36}F_3N_3O_5S$; MG 571,66

Ber.:

C 56,73 % H 6,35 %N 7,35 % S 5,61 %

Gef.:

C 56,30 % H 6,19 % N 7,39 % S 5,77%

Beispiel 28

Eine Suspension von 1,75 g 2-Methoxy-[[(4-Methoxy-3-methyl-2-pyridyl)methyl]sulfinyl]-5-(trifluormethyl)benzimidazol in 25 ml Triäthylenglykol wird unter Isobutylenatmosphäre mit 1 g Methansulfonate versetzt und während 25 Minuten bei Raumtemperatur gerührt. Das Reaktionsgemisch wird mit gesättigter wässeriger Natriumbicarbonatlösung neutralisiert; die wässerige Phase wird mehrmals mit Essigester extrahiert, und die vereinigten organischen Phasen werden über Natriumsulfat getrocknet und eingeengt. Der Rückstand wird an Kieselgel mit Aether/Methylenchlorid (20:1) und Methylenchlorid/Methanol (20:1) als Elutionsmittel chromatographiert, wobei die Methode der Mitteldruck-Flashchromatographie verwendet wird. Das harzartige Reaktionsprodukt wird im Hochvakuum getrocknet, wobei man intramolekular deprotonisiertes 2-[[[2-[2-[2-[2-(2-Hydroxyäthoxy]äthoxy]äthoxy]-2-methylpropyl]-thio]methyl]-4-methoxy-3-methyl-1-[5-(trifluormethyl)-2-benzimidazolyl]pyridiniumkation als Schaum erhält. Die Mikroanalyse zeigt folgende Werte:

Summenformel; $C_{26}H_{34}F_3N_3O_5S$•0, $8H_2O$•0,25 AcoEt MG 594,07
Ber.:
C 54,59 % H 6,38 % N 7,07 S 5,40 %
Gef.:
C 54,75 % H 6,75 % N 7,00 % S 5,52%

## Beispiel 29

Eine Suspension von 1,75 g 2-Methoxy-[[(4-Methoxy-2-pyridyl)methyl]sulfinyl]-5-(trifluormethyl)-benzimidazol in 25 ml Tetraäthylenglykol wird unter konstanter Isobutylenatmosphäre mit 1 g Methansulfonsäure versetzt und während 25 Minuten bei Raumtemperatur gerührt. Das Reaktionsgemisch wird mit gesättigter wässeriger Natriumbicarbonatlösung neutralisiert; die wässerige Phase wird mehrmals mit Essigester extrahiert, und die vereinigten organischen Phasen werden über Natriumsulfat getrocknet und eingeengt. Der Rückstand wird an Kieselgel mit Aether/Methylenchlorid (20:1) und Methylenchlorid/Methanol (20:1) als Elutionsmittel chromatographiert, wobei die Methode der Mitteldruck-Flashchromatographie verwendet wird. Das harzartige Reaktionsprodukt wird im Hochvakuum getrocknet, wobei man intramolekular deprotonisiertes 2-[[[2-[2-[2-[2-(2-Hydroxyäthoxy]äthoxy]äthoxy]äthoxy]-2-methylpropyl]thio]methyl]-4-methoxy-3-methyl-1-[5-(trifluormethyl)-2-benzimidazolyl]pyridiniumkation als Schaum erhält. Die Mikroanalyse zeigt folgende Werte:
Summenformel; $C_{28}H_{38}F_3N_3O_6S$; MG 601,68
Ber.:
C 55,89 % H 6,37 % N 6,98 S 5,33 %
Gef.:
C 55,25 % H 6,67 % N 6,43 % S 5,06%

## Beispiel 30

Eine Lösung von 62 mg gasförmigen Isobutylen in 1,75 ml Methanol und 77 mg Methansulfonsäure wird mit 93 mg 2-[[(4-Methoxy-3,5-dimethyl-2-pyridyl)methyl]sulfinly]-5-(trifluormethyl)benzimidazol versetzt, worauf man bei Raumtemperatur während 10 Minuten rührt und dann das Reaktionsgemisch mit Natriumbicarbonatlösung neutralisiert. Die Natriumbicarbonatlösung wird mehrmals mit Methylenchloride extrahiert; die vereinigten organischen Phasen werden über Natriumsulfat getrocknet und eingeengt. Der Rückstand wird aus Aether/n-Hexan im Eisschrank kristallisiert. Das erhaltene intramolekular deprotonisierte 4-Methoxy-2-[[(2-methoxy-2-methylpropyl)thio]methyl-1-[5-(trifluormethyl-2-benzimidazolyl]pyridiniumkation zeigt einen Schmelzpunkt von 90-93° (Zersetzung)

## Beispiel 31

Eine Lösung von 5 ml Cyclohexen und 1 g Methansulfonsäure in 20 ml Methanol wird mit 2 g 2-[[(4-Methoxy-3-methyl-2-pyridyl)methyl]sulfinly]-5-(trifluormethyl)benzimidazol versetzt, worauf man bei Raumtemperatur während 24 Stunden rührt und dann das Reaktionsgemisch mit einer Natriumbicarbonatlösung neutralisiert. Die Natriumbicarbonatlösung wird mehrmals mit Methylenchlorid extrahiert; die vereinigten organischen Phasen werden über Natriumsulfat getrocknet und eingeengt. Der Rückstand wird aus Aether/n-Hexan kristallisiert. Das erhaltene intramolekular deprotonisierte 4-Methoxy-2-[[trans-2-methoxycyclohexyl)thio]methyl]-3-methyl-1-[5-(trifluormethyl)-2-benzimidazolyl]pyridiniumkation zeigt einen Schmelzpunkt von 115-120° (Zersetzung)

Beispiel 32

450 mg intramolekular deprotonisiertes 2-[[2-Aethoxy-2-methylpropyl)thio]methyl]-4-methoxy-3-methyl-1-[5-(trifluormethyl)-2-benzimidazolyl]pyridiniumkation werden in Methanol gelöst und mit 110 mg Methansulfonsäure versetzt. Die Lösung wird mehrmals unter jeweiliger Zugabe von n-Hexan eingeengt. Der Rückstand wird aus tert.-Butyl-methyläther kristallisiert. Das erhaltene 2-[[(2-Aethoxy-2-methylpropyl)thio]-methyl]-4-methoxy-3-methyl-1-[5-(trifluormethyl)-2-benzimidazolyl]pyridinium-methansulfonat zeigt einen Schmelzpunkt von 49-54°.

Beispiel 33

310 mg intramolekular deprotonisiertes 2-[[(2-Aethoxy-2-methylpropyl)thio]methyl]-4-methoxy-3-methyl-1-[5-(trifluormethyl)-2-benzimidazolyl]pyridiniumkation werden in Methanol gelöst, worauf die Lösung mit 4,7N methanolischer Salzsäure angesäuert und anschliessend eingeengt wird. Zum Rückstand gibt man Essigester, engt ein, gibt nochmals Essigester zu und engt erneut ein. Man kristallisiert aus Aether und erhält 2-[[(2-Aethoxy-2-methylpropyl)thio]methyl]4-methoxy-3-methyl-1-[5-(trifluormethyl)-2-benzimidazolyl]-pyridiniumchlorid vom Schmelzpunkt 116-118°.

Beispiel 34

Eine Suspension von 380 mg 2-[[(4-Methoxy-3,5-dimethyl-2-pyridyl)methyl]sulfinyl]-5-(trifluormethyl)-benzimidazol in einer Lösung von 0,2 g Isobutylen in 3 ml tert.-Butanol wird mit 0,6 g Molekularsieb (Union Carbide Type 3A) und mit einer Lösung von 0,5g gasförmigem Chlorwasserstoff in 3,7 ml tert. Butanol versetzt. Man rührt das Reaktionsgemisch bei Raumtemperatur während 50 Minuten, giesst es anschliessend auf ein Gemisch von Eis und wässeriger Natriumbicarbonatlösung und gibt dann Methylenchlorid zu. Das Gemisch wird filtriert, und das Filtrat wird mit Methylenchlorid extrahiert. Die organische Phase wird getrocknet und eingeengt. Der Rückstand wird in 10 ml Methylenchlorid gelöst worauf man 0,7 g Silicagel (Korngrösse: 0,04-0,06 mm) zugibt und während einer Stunde bei Raumtemperatur rührt. Das Silicagel wird abfiltriert, und das Filtrat wird eingeengt. Der Rückstand wird an Silicagel mit Methylenchloride/Methanol (20:1) als Elutionsmittel chromatographiert, wobei die Methode der Mitteldruck-Flashchromatographie verwendet wird. Durch Umkristallisation aus Aether/n-Hexan erhält man intramolekular deprotonisierte 2-[[(2-Chlor-2-methylpropyl)thio]methyl]-4-methoxy-3,5-dimethyl-1-[5-(trifluormethyl-2-benzimidazolyl]-pyridiniumkation vom Schmelzpunkt 92-93°.

Beispiel 35

Eine Suspension von 100 mg intramolekular deprotonisiertem 2-[[(2-Chlor-2-methylpropyl)thio]methyl]-4-methoxy-3,5-dimethyl-1-[5-(trifluormethyl)-2-benzimidazolyl]pyridiniumkation in 2 ml 1N wässeriger Salzsäure wird während 10 Stunden bei Raumtemperatur gerührt und dann in ein Gemisch von Eis und wässeriger Natriumbicarbonatlösung gegossen. Die wässerige Phase wird mehrmals mit Methylenchlorid gewaschen; die vereinigten organischen Phasen werden getrocknet und eingeengt. Der Rückstand wird an Silicagel mit Methylenchlorid/Methanol (10:1) als Elutionsmittel chromatographiert, wobei die Methode der Mitteldruck-Flashchromatographie verwendet wird. Das harzartige Reaktionsprodukt wird im Hochvakuum getrocknet, wobei man intramolekular deportonisiertes 2-[[(2-Hydroxy-2-methylpropyl)thio]methyl]-4-methoxy-3,5-dimethyl-1-[5-(trifluormethyl)-2-benzimidazolyl]pyridiniumkation als Schaum erhält. Die Mikroanalyse zeigt folgende Werte:

Summenformel; $C_{21}H_{24}F_3N_3O_2S$; MG 439,50

Ber.:

C 57,39 % H 5,50 % N 9,56 S 7,29 %

Gef.:

C 57,15 % H 5,96 % N 9,11 % S 7,00%

Beispiel 36

a) Eine Lösung von 9,4 g 2-(Chlormethyl)-3-methyl-4-nitropyridin und 10 g 5-(Trifluormethyl)-2-benzimidazolthiol in 260 ml abs. Aceton wird mit 13 g fein gemahlenem Kalimcarbonat versetzt und bei Raumtemperatur unter Argon 18 Stunden gerührt. Im Vakuum werden in 180 ml Aceton abdestilliert, worauf der verbleibende Teil des Reaktionsgemisches auf Eis gegossen wird. Das auskristallisierte Produkt wird abfiltriert und im Trockenschrank bei 35° getrocknet. Durch Umkristallisation aus Essigester/n-Hexan erhält man 2-[[(3-Methyl-4-nitro-2-pyridyl)methyl]thio]-5-(trifluormethyl)benzimidazol vom Schmelzpunkt 192-193°.

b) 600 mg Natriumhydrid-Dispersion (55-60 % in Oel) werden in 50 ml abs. Aethanol unter Argon gelöst. Man gibt 3,68 g 2-[[(3-Methyl-4-nitro-2-pyridyl)methyl]thio]-5-(trifluormethyl)benzimidazol zu und lässt die Lösung während 1 Stunde bei 70° rühren. Man neutralisiert die Lösung mit Eisessig und dampft dann das Gemisch im Vakuum ein. Man versetzt den Rückstand mit wässeriger Natriumbicarbonatlösung und Methylenchlorid. Die organische Phase wird abgetrennt, und die wässerige Phase wird mehrmals mit Methylenchlorid extrahiert. Die vereinigten organischen Phasen werden getrocknet und eingeengt. Der Rückstand wird an Silicagel mit Methylenchlorid/Methanol (9:1) chromatographiert, wobei die Methode der Mitteldruck-Flashchromatographie verwendet und der Druck mit Stickstoffgas erzeugt wird. Man erhält 2-[[-(4-Aethoxy-3-methyl-2-pyridyl)methyl]thio]-5-(trifluormethyl)benzimidazol. Nach Umkristallisieren aus Essigester/Aether schmilzt das Produkt bei 173-177°.

c) 2-[[(4-Aethoxy-3-methyl-2-pyridyl)methyl]thio]-5-(trifluormethyl)benzimidazol kann auch wie folgt hergestellt werden:
Eine Lösung von 5 g 2-(Chlormethyl)-4-äthoxy-3-methylpyridin und 5 g 5-(Trifluormethyl)-2-benzimidazolthiol in 130 ml abs. Aceton wird mit 5 g fein gemahlenem Kaliumcarbonat versetzt und bei Raumtemperatur unter Argon 2 Stunden gerührt. Im Vakuum werden 100 ml Aceton abdestilliert, worauf der verbleibende Teil des Reaktionsgemisches auf Eis gegossen wird. Das auskristallisierte Produkt wird abfiltriert und in Methylenchlorid gelöst; die erhaltene Lösung wird mit Wasser gewaschen, getrocknet und eingeengt. Der Rückstand wird an Kieselgel unter Eluieren mit Methylenchlorid und Methylenchlorid/Essigester (1:1) chromatographiert, wobei die Methode der Mitteldruck-Flashchromatographie verwendet wird. Man erhält 2-[[(4-Aethoxy-3-methyl-2-pyridyl)methyl]thio]-5-(trifluormethyl)benzimidazol, welches nach Umkristallisieren aus Essigester/Aether bei 173-177° schmilzt.

d) Eine Lösung von 2,5 g 2-[[(4-Aethoxy-3-methyl-2-pyridyl)methyl]thio]-5-(trifluormethyl)benzimidazol in 20 ml Chloroform wird unter Argon bei -40° schnell mit einer Lösung von 1,5 g m-Chlorperbenzoesäure in Chloroform versetzt. Anschliessend wird die Lösung 10 Minuten gerührt und mit 10-prozentiger Natriumcarbonatlösung extrahiert; die Chloroformlösung wird mit 3 Tropfen Triäthylamin versetzt, getrocknet und eingeengt. Das erhaltene 2-[[(4-Aethoxy-3-methyl-2-pyridyl)methyl]sulfinyl]-5-(trifluormethyl)-2-benzimidazol wird direkt weiterverarbeitet.

e) 80 ml Aethanol, gesättigt mit Isobutylen, wird mit 2,7 g 2-[[(4-Aethoxy-3-methyl-2-pyridyl)methyl]-sulfinyl]-5-(trifluormethyl)-2-benzimidazol versetzt, und unter Isobutylenatmosphäre wird eine Lösung von 1 g Methansulfonsäure in 5 ml Aethanol zugegeben. Man rührt die Lösung während 18 Stunden bei Raumtemperatur und engt dann ein. Der Rückstand wird mit Methylenchlorid und wässeriger Natriumcarbonatlösung versetzt: die organische Phase wird abgetrennt, und die wässrige Phase wird mit Methylenchlorid gewaschen. Die vereinigten organischen Phasen werden getrocknet und eingeengt. Der Rückstand wird an Kieselgel unter Eluieren mit Methylenchlorid/Methanol (10:1) chromatographiert, wobei die Methode der Mitteldruck-Flashchromatographie verwendet wird. Das erhaltene intramolekular deprotonisierte 4-Aethoxy-2-[[(2-äthoxy-2-methylpropyl)thio]methyl]-1-[5-(trifluormethyl)-2-benzimidazolyl]pyridiniumkation wird in Aether mit gasförmigen Chlorwasserstoff angesäuert, worauf man die Lösung einengt und den Rückstand aus Aether kristallisiert. Man erhält 4-Aethoxy-2-[[(2-äthoxy-2-methylpropyl)thio]methyl]-1-[5-(trifluormethyl)-2-benzimidazolyl]pyridiniumchlorid, welches bei 145-147° unter Zersetzung schmilzt.

Beispiel 37

a) Eine Suspension von 13 g 5,6-Dichlor-2-benzimidazolthiol in 360 ml Alkohol wird mit 13 g 2-Chlormethyl-4-methoxy-3-methylpyridin-hydrochlorid versetzt. Unter Eiskühlung tropft man eine Lösung von 4 g Natriumhydroxid in 170 ml Wasser zu, lässt das Gemisch über Nacht am Rückfluss kochen und engt es

dann im Vakuum auf ca. 1/3 seines Volumens ein. Nach Zugabe von 600 ml Wasser werden die Kristalle abfiltriert und hierauf zuerst mit Wasser und dann mit Aether gründlich gewaschen. Man erhält 5,6-Dichlor-2-[[(4-methoxy-3-methyl-2-pyridyl)methyl]thio]benzimidazol vom Schmelzpunkt 254-256°.

b) Eine Lösung von 1,0 g 5,6-Dichlor-2-[[(4-methoxy-3-methyl-2-pyridyl)methyl]thio]benzimidazol in 150 ml Methylenchlorid und 30 ml Methanol wird mit 0,5 g Kaliumcarbonat versetzt. Bei -30° gibt man unter Rühren 0,6 g m-Chlorperbenzoesäure zu, worauf die Lösung 5 Minuten weitergerührt und anschliessend in ein Gemisch von 20 ml gesättigter Natriumbicarbonatlösung und 20 ml Wasser ge gossen wird. Die abgetrennte organische Phase wird über Natriumsulfat getrocknet, mit 0,5 ml Triäthylamin versetzt und im Vakuum eingedampft. Kristallisation aus Methylenchlorid-Methanol-Aether ergibt 5,6-Dichlor-2-[[(4-methoxy-3-methyl-2-pyridyl)methyl]sulfinyl]benzimidazol vom Schmelzpunkt 173°.

c) Eine Lösung von 2 g Methansulfonsäure in 200 ml Aethanol wird 45 Minuten mit Isobutylengas gesättigt, dann mit 3,7 g 5,6-Dichlor-2-[[(4-methoxy-3-methyl-2-pyridyl)methyl]sulfinyl]benzimidazol versetzt, über Nacht bei Raumtemperatur gerührt und anschliessend im Vakuum eingdampft. Der Rückstand wird in Methylenchlorid aufgenommen, worauf die Lösung mit 100 ml gesättigter Natriumbicarbonatlösung extrahiert, mit Wasser neutralgewaschen, getrocknet und im Vakuum eingeengt wird. Kristallisation des Rückstandes aus Methylenchlorid/Petroläther (tiefs.) ergibt intramolekular deprotonisiertes 1-(5,6-Dichlor-2-benzimidazolyl)-2-[[(2-äthoxy-2-methylpropyl)thio]methyl]-4-methoxy-3-methylpyridiniumkation vom Schmelzpunkt 141-143°.

## Beispiel 38

Eine Lösung von 3,7 g 5,6-Difluor-2-[[(4-methoxy-3-methyl-2-pyridyl)methyl[sulfinyl]benzimidazol in 200 ml tert. Butanol.wird 45 Minuten mit Isobutylengas gesättigt, dann mit einer frisch zubereiteten Lösung von 23 g Chlorwasserstoff-Gas in 200 ml tert. Butanol versetzt, 48 Stunden bei Raumtemperatur gerührt und anschliessend im Vakuum eingedampft. Der Rückstand wird in Methylenchlorid aufgenommen, worauf die Lösung mit 100 ml gesättigter Natriumbicarbonatlösung extrahiert, mit Wasser neutralgewaschen, getrocknet und im Vakuum eingeengt wird. Kristallisation des Rückstandes aus Methylenchlorid/Petroläther (tiefs.) ergibt intramolekular deprotonisiertes 2-[[(2-Chlor-2-methylpropyl)thio]methyl]-1-(5,6-dichlor-2-benzimidazolyl]-4-methoxy-3-methylpyridiniumkation vom Schmelzpunkt 136-138°.

## Beispiel 39

Eine Suspension von 2,7 g 2-[[(4-Aethoxy-3-methyl-2-pyridyl)methyl]sulfinyl]-5-(trifluormethyl)-benzimidazol in einer Lösung von 1,4 g Isobutylen in 21 ml tert.-Butanol wird mit 2 g Molekularsieb (Union Carbide Type 3A) und mit einer Lösung von 3,5g gasförmigem Chlorwasserstoff in 26 ml tert. Butanol versetzt. Das Reaktionsgemisch wird bei Raumtemperatur während 50 Minuten, geruht und anschliessend auf ein Gemisch von Eis und 200 ml wässeriger Natriumbicarbonatlösung gegossen, worauf man Methylenchlorid zugibt. Der unlösliche Teil des Gemisches wird über Kieselgel abfiltriert, und das Filtrat wird mehrmals mit Methylenchlorid extrahiert. Die organische Phasen werden vereinigt, mit Natriumsulfat getrocknet, filtriert und eingeengt. Der Rückstand wird in 80 ml Methylenchlorid gelöst worauf man 5 g Kieselgel (Korngrösse: 0,04-0,06 mm) zugibt und während einer Stunde bei Raumtemperatur rührt. Man filtriert das Kieselgel ab, engt das Filtrat ein und kristallisiert den Rückstand aus Aether. Das erhaltene intramolekular deprotonisierte 2-[[(2-Chlor-2-methylpropyl)thio]methyl]-4-äthoxy-3-methyl-1-[5-(trifluormethyl)-2-benzimidazolyl]pyridiniumkation zeigt einen Schmelzpunkt von 152° (Zersetzung).

## Beispiel 40

Eine Suspension von 1,5 g intramolekular deprotonisiertem 2-[[(2-Chlor-2-methylpropyl)thio]methyl]-4-äthoxy-3-methyl-1-[5-(trifluormethyl)-2-benzimidazolyl]pyridiniumkation in 30 ml 1N wässeriger Salzsäure wird 9 Stunden bei Raumtemperatur gerührt und dann auf ein Gemisch von Eis und wässeriger Natriumbicarbonatlösung gegossen, worauf man mehrmals mit Methylenchlorid extrahiert. Die vereinigten Methylenchloridextrakte werden mit Natriumsulfat getrocknet, filtriert und eingeengt. Der Rückstand wird an Kieselgel

mit Methylenchlorid/Methanol (20:1) als Elutionsmittel chromatographiert, wobei die Methode der Mitteldruck-Flashchromato graphie verwendet wird. Durch Kristallisation aus Aether erhalt man intramolekular deportonisiertes 2-[[(2-Hydroxy-2-methylpropyl)thio]methyl]-4-äthoxy-3-methyl-1-[5-(trifluormethyl)-2-benzimidazolyl]pyridiniumkation von Schmelzpunkt 162° (Zersetzung).

Beispiel 41

Eine Lösung von 1,3 g intramolekular deprotonisiertem 2-[[(2-Hydroxy-2-methylpropyl)thio]methyl]-4-äthoxy-3-methyl-1-[5-trifluormethyl)-2-benzimidazolyl]pyridiniumkation in 4,1 ml Essigsäure und 8,3 ml Acetanhydrid wird mit 8 Tropfen Perchlorsäure versetzt und während 5 Stunden bei Raumtemperatur gerührt. Man neutralisiert das Reaktionsgemisch mit gesättigter wässeriger Natriumcarbonatlösung, extrahiert mehrmals mit Aether, trocknet die Extrakte über Natriumsulfat, filtriert und dampft den Aether ab. Der Rückstand wird aus Aether/n-Hexan kristallisiert, wobei man intramolekular deprotonisiertes 2-[[(2-Acetoxy-2-methylpropyl)thio]methyl]-4-äthoxy-3-methyl-1-[5-(trifluormethyl)-2-benzimidazolyl]pyridiniumkation vom Schmelzpunkt 108-109° (Zersetzung) erhält.

Beispiel 42

Eine Lösung von 2 g intramolekular deprotonisiertem 2-[[[2-(2-Hydroxyäthoxy)-2-methylpropyl)thio]-methyl]-4-methoxy-3-methyl-1-[5-(trifluormethyl)-2-benzimidazolyl]pyridiniumkation in 6,2 ml Essigsäure und 12,5 ml Acetanhydrid wird mit 8 Tropfen Perchlorsäure versetzt und während 5 Stunden bei Raumtemperatur gerührt. Man neutralisiert das Reaktionsgemisch mit gesättigter wässeriger Sodalösung, extrahiert mehrmals mit Methylenchlorid, trocknet die Extrakte über Natriumsulfat, filtriert und dampft das Methylenchlorid ab. Der Rückstand wird an Kieselgel mit Methylenchlorid/Methanol (10:1) als Elutionsmittel chromatographiert, wobei die Methode der Mitteldruck-Flash chromatographie verwendet wird. Durch Kristallisation aus Aether/n-Hexan erhält man intramolekular deprotonisiertes 2-[[[2-(2-Acetoxyäthoxy)-2-methylpropyl]-thio]methyl]-4-methoxy-3-methyl-1-[5-(trifluormethyl)-2-benzimidazolyl]pyridiniumkation vom Schmelzpunkt 85-89°.

Beispiel 43

Eine Lösung von 1,3 g Methansulfonsäure und etwa 8 g gasförmigem Isobutylen in 100 ml Isopropanol wird mit 2,5 g 2-[[(4-Methoxy-3-methyl-2-pyridyl)methyl]sulfinyl]-5-(trifluormethyl)benzimidazol versetzt, worauf man bei Raumtemperatur unter Isobutylenatmosphere während 66 Stunden rührt und dann das Reaktionsgemisch eindampft. Man versetzt den Rückstand mit Methylenchlorid und wässeriger Natriumbicarbonatlösung, trennt die Methylenchloridphase ab und extrahiert die wässerige Phase mit Methylenchlorid. Die vereinigten organischen Phasen werden getrocknet und eingeengt. Der Rückstand wird an Kieselgel mit Methylenchlorid/Methanol (20:1) als Elutionsmittel chromatographiert, wobei die Methode der Mitteldruck-Flashchromatographie verwendet wird. Das gereinigte Reaktionsprodukt wird in Essigester gelöst und mit einer Lösung von gasförmiger Salzsäure in Aethanol angesäuert, worauf man das Lösungsmittelsystem abdampft. Der Rückstand wird aus Essigester/Aether kristallisiert, wobei man 2-(2-Isopropoxy-2-methylpropyl)-4-methoxy-3-methyl-1-[5-(trifluormethyl)-2-benzimidazolyl]pyridiniumchlorid vom Schmelzpunkt 123-126° erhält.

Beispiel A

Kristalline Verbindungen der Formel I können als Wirkstoffe zur Herstellung von Hartgelatinekapseln verwendet werden, deren Inhalt pro Kapsel folgende Zusammensetzung aufweist:

| | |
|---|---|
| Wirkstoff | 50,0 mg |
| Milchzucker pulv. | 40,0 mg |
| Milchzucker krist. | 130,0 mg |
| Maisstärke weiss | 20,0 mg |
| Talk | 8,0 mg |
| Magnesiumstearat | 2,0 mg |
| Füllgewicht pro Kapsel | 250,0 mg |

Der Wirkstoff und die Hilfsstoffe werden miteinander vermischt, und das Gemisch wird in Hartgelatinekapseln geeigneter Grösse abgefüllt. Erforderlichenfalls werden die Kapseln anschliessend mit einem magensaftresistenten Lack, bestehend aus Hydroxypropylmethylcellulosephthalat, versehen.

**Ansprüche**

1. Benzimidazol-2-yl-pyridiniumverbindungen der allgemeinen Formel

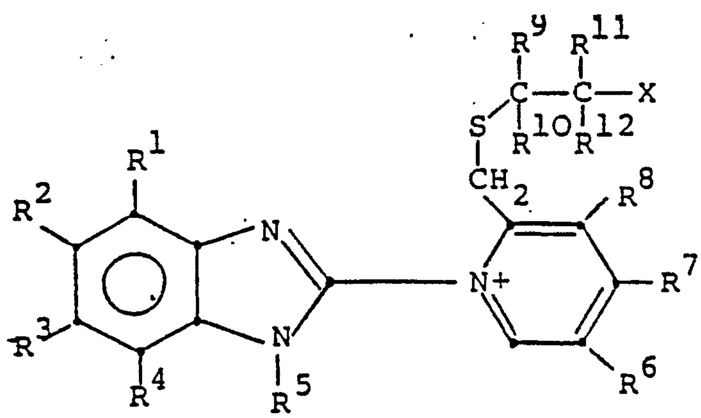

I

worin
- $R^1$, $R^2$, $R^3$ und $R^4$ je Wasserstoff, Fluor, Chlor, Trifluormethyl, Cyano oder einen Rest der Formel

$$-COOR^{13} \quad (a), \quad -CONR^{14}R^{15} \quad (b), \quad -SOR^{16} \quad (c) \quad \text{oder} \quad -SO_2R^{16} \quad (d)$$

oder zwei benachbarte dieser Substituenten zusammen mit den Kohlenstoffatomen, an welchen sie gebunden sind, einen mindestens eines der Strukurelemente

$$-CO- \quad (f), \quad -COO- \quad (g), \quad -CON(R^{14})- \quad (h), \quad -SO- \quad (i) \quad \text{oder} \quad -SO_2- \quad (j)$$

enthaltenden 5-, 6- oder 7-gliedrigen Ring bedeuten, mit der Massgabe, dass von den Symbolen $R^1$, $R^2$, $R^3$ und $R^4$ mindestens eines, höchstens aber drei Wasserstoff bedeuten;
- $R^5$ Wasserstoff oder eine negative Ladung bedeutet;
- $R^6$ und $R^8$ je Wasserstoff oder niederes Alkyl bedeuten;

- $R^7$ niederes Alkoxy bedeutet;
- $R^9$ und $R^{10}$ je Wasserstoff oder niederes Alkyl und $R^{11}$ und $R^{12}$ je niederes Alkyl bedeuten; oder zwei der Substituenten $R^9$ und $R^{10}$ oder $R^9$ und $R^{11}$ oder $R^{11}$ und $R^{12}$ zusammen mit dem oder den Kohlenstoffatom(en), an welche(s) sie gebunden sind, einen 5-, 6- oder 7-gliedrigen carbocyclischen Ring und die beiden übrigen der Substituenten $R^9$, $R^{10}$, $R^{11}$ und $R^{12}$ je Wasserstoff oder niederes Alkyl bedeuten; oder $R^9$ zusammen mit X eine zusätzliche Kohlenstoff-Kohlenstoff-Bindung, $R^{10}$ Wasserstoff oder niederes Alkyl und $R^{11}$ und $R^{12}$ je niederes Alkyl bedeuten;
- $R^{13}$ niederes Alkyl bedeutet;
- $R^{14}$ und $R^{15}$ je Wasserstoff oder niederes Alkyl oder zusammen mit dem Stickstoffatom einen 5-, 6- oder 7-gliedrigen gesättigten heterocyclischen Ring bedeuten;
- $R^{16}$ niederes Alkyl bedeutet;
- X Chlor, Brom oder einen Rest der Formel $-OR^{17}$ bedeutet oder, wie schon erwähnt, zusammen mit $R^9$ eine zusätzliche Kohlenstoff-Kohlenstoff-Bindung bedeutet;
- $R^{17}$ Wasserstoff, Acyl oder einen Rest der Formel

$$-CH(R^{18})R^{19}$$

(k)

bedeutet;
- $R^{18}$ Wasserstoff oder niederes Alkyl bedeutet; und
- $R^{19}$ Wasserstoff, niederes Alkyl, niederes Alkenyl, niederes Hydroxyalkyl, niederes Alkoxy-niederes-Alkyl, Hydroxy-niederes-Alkoxy-niederes-Alkyl, niederes Alkoxy-niederes-Alkoxy-niederes-Alkyl, Hydroxy-niederes-Alkoxy-niederes-Alkoxy-niederes-Alkyl, niederes Alkoxy-niederes-Alkoxy-niederes-Alkoxy-niederes-Alky, Hydroxy-niederes-Alkoxy-niederes-Alkoxy-niederes-Alkoxy-niederes-Alkyl, Acyloxy-niederes-Alkyl oder Acyloxy-niederes-Alkoxy-niederes-Alkyl bedeutet;

wobei das Molekül gesamthaft ungeladen oder einfach positiv geladen ist und wobei im letzteren Fall ein externes Anion vorliegt.

2. Verbindungen gemäss Anspruch 1, worin keines der Symbole $R^1$ bis $R^4$ Chlor bedeutet.

3. Verbindungen gemäss Anspruch 1, worin $R^1$ und $R^4$ je Wasserstoff und entweder $R^2$ und $R^3$ je Fluor oder je Chlor oder zusammen einen Rest der Formel $-C(CH_3)_2-CO-C(CH_3)_2-$ oder $R^2$ Fluor oder Trifluromethyl und $R^3$ Wasserstoff bedeuten.

4. Verbindungen gemäss Anspruch 3, worin $R^2$ Trifluormethyl und $R^3$ Wasserstoff bedeuten.

5. Verbindungen gemäss einem der Ansprüche 1 bis 4, worin $R^6$ Wasserstoff oder Methyl, $R^7$ Methoxy oder Aethoxy und $R^8$ Methyl bedeuten.

6. Verbindungen gemäss Anspruch 5, worin $R^7$ Methoxy bedeutet.

7. Verbindungen gemäss einem der Ansprüche 1 bis 6, worin entweder $R^9$ und $R^{10}$ je Wasserstoff, $R^{11}$ und $R^{12}$ je Methyl und X Chlor, Hydroxy, Methoxy, Aethoxy, Propoxy, Butoxy, Acetoxy, 2-Hydroxyäthoxy, 2-Methoxyäthoxy 2-(2-Hydroxyäthoxy)äthoxy, 2-(2-Methoxyäthoxy)äthoxy, 2-[2-(2-Hydroxyäthoxy)äthoxy]-äthoxy oder 2-[2-(2-Methoxyäthoxy)äthoxy]äthoxy bedeuten oder $R^9$ zusammen mit X eine zusätzliche Kohlenstoff-Kohlenstoff-Bindung, $R^{10}$ Wasserstoff und $R^{11}$ und $R^{12}$ je Methyl bedeuten oder $R^9$ und $R^{11}$ zusammen Tetramethylen, $R^{10}$ und $R^{12}$ je Wasserstoff und X Methoxy bedeuten.

8. Verbindungen gemäss Anspruch 7, worin entweder $R^9$ und $R^{10}$ je Wasserstoff, $R^{11}$ und $R^{12}$ je Methyl und X Chlor, Hydroxy, Methoxy, Aethoxy, Propoxy, Acetoxy, 2-Hydroxyäthoxy oder 2-Methoxyäthoxy bedeuten oder $R^9$ zusammen mit X eine zusätzliche Kohlenstoff-Kohlenstoff-Bindung, $R^{10}$ Wasserstoff und $R^{11}$ und $R^{12}$ je Methyl bedeuten.

9. Verbindungen gemäss Anspruch 8, worin $R^9$ und $R^{10}$ je Wasserstoff, $R^{11}$ und $R^{12}$ je Methyl und X Aethoxy, Acetoxy, 2-Hydroxyäthoxy oder 2-Methoxyäthoxy bedeuten.

10. 2-[[[2-(2-Hydroxyäthoxy)-2-methylpropyl]thio]methyl]-4-methoxy-3-methyl-1-[5-(trifluormethyl)-2-benzimidazolyl]pyridinium-methansulfonat.

11. Intramolekular deprotonisiertes 2-[[[2-(2-Hydroxyäthoxy)-2-methylpropyl]thio]methyl]-4-methoxy-3-methyl-1-[5-(trifluormethyl)-2-benzimidazolyl]pyridiniumkation.

12. 4-Methoxy-2-[[[2-(2-methoxyäthoxy)-2-methylpropyl]thio]methyl]-3-methyl-1-[5-(trifluormethyl)-2-benzimidazolyl]pyridinium-methansulfonat.

13. Intramolekular deprotonisiertes 4-Methoxy-2-[[[2-(2-methoxyäthoxy)-2-methylpropyl]thio]methyl]-3-methyl-1-[5-(trifluormethyl)-2-benzimidazolyl]pyridiniumkation.

14. 2-[[(2-Acetoxy-2-methylpropyl)thio]methyl]-4-methoxy-3-methyl-1-[5-(trifluormethyl)-2-benzimidazolyl]pyridinium-methansulfonat.

15. 2-[[(2-Acetoxy-2-methylpropyl)thio]methyl]-4-methoxy-3-methyl-1-[5-(trifluormethyl)-2-benzimidazolyl]pyridiniumchlorid.

16. Intramolekular deprotonisiertes 2-[[(2-Acetoxy-2-methylpropyl)thio]methyl]-4-methoxy-3-methyl-1-[5-(trifluormethyl)-2-benzimidazolyl]pyridiniumkation.

17. 2-[[(2-Aethoxy-2-methylpropyl)thio]methyl]-4-methoxy-3-methyl-1-[5-(trifluormethyl)-2-benzimidazolyl]pyridinium-methansulfonat.

18. 2-[[(2-Aethoxy-2-methylpropyl)thio]methyl]-4-methoxy-3-methyl-1-[5-(trifluormethyl)-2-benzimidazolyl]pyridiniumchlorid.

19. Intramolekular deprotonisiertes 2-[[(2-Aethoxy-2-methylpropyl)thio]methyl]-4-methoxy-3-methyl-1-[5-(trifluormethyl)-2-benzimidazolyl]pyridiniumkation.

20. Intramolekular deprotonisiertes 2-[[(2-Chlor-2-methylpropyl)thio]methyl]-4-methoxy-3-methyl-1-[5-(trifluormethyl)-2-benzimidazolyl]pyridiniumkation;
2-[[(2-Chlor-2-methylpropyl)thio]methyl]-4-methoxy-3-methyl-1-[5-(trifluormethyl)-2-benzimidazolyl]-pyridiniumchlorid;
intramolekular deprotonisiertes 2-[[(2-Hydroxy-2-methylpropyl)thio]methyl]-4-methoxy-3-methyl-1-[5-(trifluormethyl)-2-benzimidazolyl]pyridiniumkation;
intramolekular deprotonisiertes 4-Methoxy-2-[[(2-methoxy-2-methylpropyl)thio]methyl]-3-methyl-1-[5-(trifluormethyl)-2-benzimidazolyl]pyridiniumkation;
intramolekular deprotonisiertes 4-Methoxy-3-methyl-2-[[(2-methylpropyl)thio]methyl]-1-[5-(trifluormethyl)-2-benzimidazolyl]pyridiniumkation;
intramolekular deprotonisiertes 4-Methoxy-3-methyl-2-[[(2-methyl-2-propoxypropyl)thio]methyl]-1-[5-(trifluormethyl)-2-benzimidazolyl]pyridiniumkation;
4-Methoxy-3-methyl-2-[[(2-methylpropyl)thio]methyl]-1-[5-(trifluormethyl)-2-benzimidazolyl]pyridiniumchlorid;
und
intramolekular deprotonisiertes 2-[[(2-Hydroxy-2-methylpropyl)thio]methyl]-4-methoxy-3,5-dimethyl-1-[5-(trifluormethyl)-2-benzimidazolyl]pyridniumkation.

21. Verbindungen gemäss einem der Ansprüche 1 bis 20 zur Anwendung als therapeutische Wirkstoffe.

22. Verbindungen gemäss einem der Ansprüchen 1 bis 20 zur Anwendung als therapeutische Wirkstoffe mit magensäuresekretionshemmenden und/oder mucosaprotektiven Eigenschaften bei der Bekämpfung oder Verhütung von Ulcus ventriculi und/oder duodeni.

23. Verfahren zur Herstellung von Verbindungen gemäss einem der Ansprüche 1 bis 20, dadurch gekennzeichnet, dass man
a) eine Verbindung der allgemeinen Formel

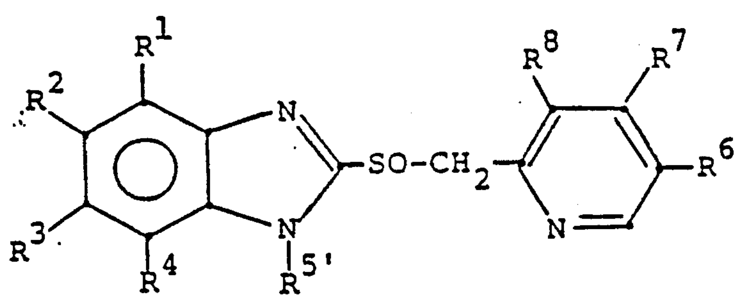
II

worin $R^1$, $R^2$, $R^3$, $R^4$, $R^6$, $R^7$ und $R^8$ die in Anspruch 1 definierte Bedeutung besitzen und $R^{5'}$ Wasserstoff bedeutet,
unter saueren Bedingungen mit einer Verbindung der allgemeinen Formel

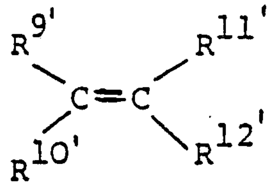

III

worin $R^{9'}$ und $R^{10'}$ je Wasserstoff oder, niederes Alkyl und $R^{11'}$ und $R^{12'}$ je niederes Alkyl bedeuten, oder zwei der Substituenten $R^9$ und $R^{10}$ oder $R^9$ und $R^{11}$ und $R^{12}$ zusammen mit dem oder den Kohlenstoffatom(en), an welche(s) sie gebunden sind, einen 5-, 6- oder 7-gliedrigen carbocyclischen Ring und die beiden übrigen der Substituenten $R^9$, $R^{10}$ $R^{11}$ und $R^{12}$ je Wasserstoff oder niederes Alkyl bedeuten,
und einer Verbindung der allgemeinen Formel

$HX'$ IV

worin $X'$ Chlor, Brom oder einen Rest der Formel $-OR^{17}$ und $R^{17}$ Wasserstoff oder einen Rest der in Anspruch 1 definierten Formel (k) bedeuten,
umsetzt; oder
b) eine Verbindung der Formel I, worin X Chlor oder Brom bedeutet, mit einer Verbindung der allgemeinen Formel

$HOR^{17'}$ V

worin $R^{17'}$ Wasserstoff oder einen Rest der in Anspruch 1 definierten Formel (k) bedeutet,
umsetzt; oder
c) eine Verbindung der Formel I, worin X einen Rest der Formel $-OR^{17}$ und $R^{17}$ Wasserstoff bedeuten, dehydratisiert; oder
d) eine Verbindung der Formel I, worin X einen Rest der Formel $-OR^{17}$, $R^{17}$ Wasserstoff oder einen Rest der in Anspruch 1 definierten Formel (k) und $R^{19}$ niederes Hydroxyalkyl oder Hydroxy-niederes Alkoxy-niederes-Alkyl bedeuten, acyliert;
worauf man das erhaltene Produkt als Salz oder inneres Salz isoliert und erwünschtenfalls ein inneres Salz in ein pharmazeutisch annehmbares Salz überführt.

24. Arzneimittel, enthaltend eine Verbindung gemäss einem der Ansprüche 1 bis 20 und ein therapeutisch inertes Excipiens.

25. Arzneimittel gemäss Anspruch 24 mit magensäuresekretions-hemmenden und/oder mucosaprotektiven Eigenschaften zur Bekämpfung oder Verhütung von Ulcus ventriculi und/oder duodeni.

26. Verwendung von Verbindungen gemäss einem der Ansprüche 1 bis 20 bei der Bekämpfung oder Verhütung von Krankheiten, insbesondere bei der Bekämpfung oder Verhütung von Ulcus ventriculi und/oder duodeni bzw. zur Herstellung von Arzneimitteln gegen Ulcus ventriculi und/oder duodeni.

Patentansprüche für die folgenden Vertragsstaaten: ES, GR

1. Verfahren zur Herstellung von Benzimidazol-2-yl-pyridiniumverbindungen der allgemeinen Formel

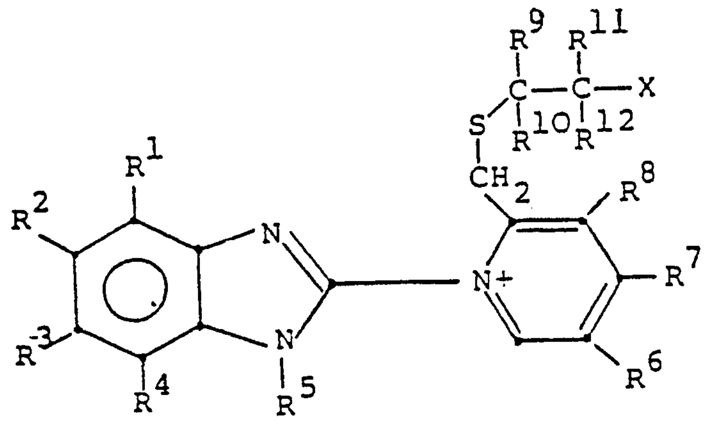

I

worin

- $R^1$, $R^2$, $R^3$ und $R^4$ je Wasserstoff, Fluor, Chlor, Trifluormethyl, Cyano oder einen Rest der Formel

$-COOR^{13}$ , $-CONR^{14}R^{15}$ , $-SOR^{16}$ oder $-SO_2R^{16}$

(a) (b) (c) (d)

oder zwei benachbarte dieser Substituenten zusammen mit den Kohlenstoffatomen, an welchen sie gebunden sind, einen mindestens eines der Strukturelemente

$-CO-$ , $-COO-$ , $-CON(R^{14})-$ , $-SO-$ oder $-SO_2-$

(f) (g) (h) (i) (j)

enthaltenden 5-, 6- oder 7-gliedrigen Ring bedeuten, mit der Massgabe, dass von den Symbolen $R^1$, $R^2$, $R^3$ und $R^4$ mindestens eines, höchstens aber drei Wasserstoff bedeuten;

- $R^5$ Wasserstoff oder eine negative Ladung bedeutet;
- $R^6$ und $R^8$ je Wasserstoff oder niederes Alkyl bedeuten;
- $R^7$ niederes Alkoxy bedeutet;
- $R^9$ und $R^{10}$ je Wasserstoff oder niederes Alkyl und $R^{11}$ und $R^{12}$ je niederes Alkyl bedeuten; oder zwei der Substituenten $R^9$ und $R^{10}$ oder $R^9$ und $R^{11}$ oder $R^{11}$ und $R^{12}$ zusammen mit dem oder den Kohlenstoffatom(en), an welche(s) sie gebunden sind, einen 5-, 6- oder 7-gliedrigen carbocyclischen Ring und die beiden übrigen der Substituenten $R^9$, $R^{10}$, $R^{11}$ und $R^{12}$ je Wasserstoff oder niederes Alkyl bedeuten; oder $R^9$ zusammen mit X eine zusätzliche Kohlenstoff-Kohlenstoff-Bindung, $R^{10}$ Wasserstoff oder niederes Alkyl und $R^{11}$ und $R^{12}$ je niederes Alkyl bedeuten;
- $R^{13}$ niederes Alkyl bedeutet;
- $R^{14}$ und $R^{15}$ je Wasserstoff oder niederes Alkyl oder zusammen mit dem Stickstoffatom einen 5-, 6- oder 7-gliedrigen gesättigten heterocyclischen Ring bedeuten;
- $R^{16}$ niederes Alkyl bedeutet;
- X Chlor, Brom oder einen Rest der Formel $-OR^{17}$ bedeutet oder, wie schon erwähnt, zusammen mit $R^9$ eine zusätzliche Kohlenstoff-Kohlenstoff-Bindung bedeutet;
- $R^{17}$; Wasserstoff, Acyl oder einen Rest der Formel

$$-CH(R^{18})R^{19}$$

(k)

bedeutet;

- $R^{18}$ Wasserstoff oder niederes Alkyl bedeutet; und
- $R^{19}$ Wasserstoff, niederes Alkyl, niederes Alkenyl, niederes Hydroxyalkyl, niederes Alkoxy-niederes-Alkyl, Hydroxy-niederes-Alkoxy-niederes-Alkyl, niederes Alkoxy-niederes-Alkoxy-niederes-Alkyl, Hydroxy-niederes-Alkoxy-niederes-Alkoxy-niederes-Alkyl, niederes Alkoxy-niederes-Alkoxy-niederes-Alkoxy-niederes-Alkyl, Hydroxy-niederes-Alkoxy-niederes-Alkoxy-niederes-Alkoxy-niederes-Alkyl, Acyloxy-niederes-Alkyl oder Acyloxy-niederes-Alkoxy-niederes-Alkyl bedeutet;

wobei das Molekül gesamthaft ungeladen oder einfach positiv geladen ist und wobei im letzteren Fall ein externes Anion vorliegt, dadurch gekennzeichnet, dass man

a) eine Verbindung der allgemeinen Formel

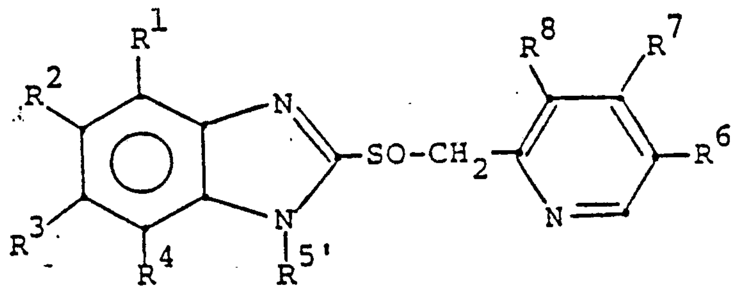

II

worin $R^1$, $R^2$, $R^3$, $R^4$, $R^6$, $R^7$ und $R^8$ obige Bedeutung besitzen und $R^{5'}$ Wasserstoff bedeutet, unter sauren Bedingungen mit einer Verbindung der allgemeinen Formel

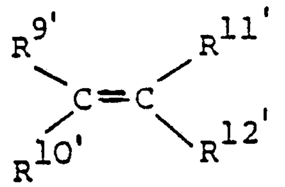

III

worin $R^{9'}$ und $R^{10'}$ je Wasserstoff oder niederes Alkyl und $R^{11'}$ und $R^{12'}$ je niederes Alkyl bedeuten, oder zwei der Substituenten $R^{9'}$ und $R^{10'}$ oder $R^{9'}$ und $R^{11'}$ oder $R^{11'}$ und $R^{12'}$ zusammen mit dem oder den Kohlenstoffatom(en), an welche(s) sie gebunden sind, einen 5-, 6- oder 7-gliedrigen carbocyclischen Ring und die beiden übrigen der Substituenten $R^{9'}$, $R^{10'}$ $R^{11'}$ und $R^{12'}$ je Wasserstoff oder niederes Alkyl bedeuten,

und einer Verbindung der allegemeinen Formel

$HX'$ IV

worin $X'$ Chlor, Brom oder einen Rest der Formel $-OR^{17}$ und $R^{17}$ Wasserstoff oder einen Rest der obige Formel (k) bedeuten,

umsetzt; oder

b) eine Verbindung der Formel I, worin X Chlor oder Brom bedeutet, mit einer Verbindung der allgemeinen Formel

$HOR^{17'}$ V

worin $R^{17'}$ Wasserstoff oder einen Rest der obigen Formel (k) bedeutet,

umsetzt; oder

c) eine Verbindung der Formel I, worin X einen Rest der Romel $-OR^{17}$ und $R^{17}$ Wasserstoff bedeuten, dehydratisiert; oder

d) eine Verbindung der Formel I, worin einen Rest der Formel $-OR^{17}$, $R^{17}$ Wasserstoff oder einen Rest der obigen Formel (k) und $R^{19}$ niederes Hydroxyalkyl oder Hydroxy-niederes Alkoxy-niederes-Alkyl bedeuten, acyliert;

worauf man das erhaltene Produkt als Salz oder inneres Salz isoliert und erwünschtenfalls ein inneres Salz in ein pharmazeutisch annehmbares Salz überführt.

2. Verfahren gemäss Anspruch 1, dadurch gekennzeichnet, dass man eine Verbindung der in Anspruch 1 definierten Formel I herstellt, worin keines der Symbole $R^1$ bis $R^4$ Chlor bedeutet.

3. Verfahren gemäss Anspruch 1, dadurch gekennzeichnet, dass man eine Verbindung der in Anspruch 1 definierten Formel I herstellt, worin $R^1$ und $R^4$ je Wasserstoff und entweder $R^2$ und $R^3$ je Fluor oder je Chlor oder zusammen einen Rest der Formel $-C(CH_3)_2-CO-C(CH_3)_2-$ oder $R^2$ Fluor oder Trifluormethyl und $R^3$ Wasserstoff bedeuten.

4. Verfahren gemäss Anspruch 3, dadurch gekennzeichnet, dass man eine Verbindung der in Anspruch 1 definierten Formel I herstellt, worin $R^2$ Trifluormethyl und $R^3$ Wasserstoff bedeuten.

5. Verfahren gemäss einem der Ansprüche 1 bis 4, dadurch gekennzeichnet, dass man eine Verbindung der in Anspruch 1 definierten Formel I herstellt, worin $R^6$ Wasserstoff oder Methyl, $R^7$ Methoxy oder Aethoxy und $R^8$ Methyl bedeuten.

6. Verfahren gemäss Anspruch 5, dadurch gekennzeichnet, dass man eine Verbindung der in Anspruch 1 definierten Formel I herstellt, worin $R^7$ Methoxy bedeutet.

7. Verfahren gemäss einem der Ansprüche 1 bis 6, dadurch gekennzeichnet, dass man eine Verbindung der in Anspruch 1 definierte Formel I herstellt, worin entweder $R^9$ und $R^{10}$ je Wasserstoff, $R^{11}$ und $R^{12}$ je Methyl und X Chlor, Hydroxy, Methoxy, Aethoxy, Propoxy, Butoxy, Acetoxy, 2-Hydroxyäthoxy, 2-Methoxyäthoxy 2-(2-Hydroxyäthoxy)äthoxy, 2-(2-Methoxyäthoxy)äthoxy, 2-[2-(2-Hydroxyäthoxy)äthoxy]-äthoxy oder 2-[2-(2-Methoxyäthoxy)äthoxy]äthoxy bedeuten oder $R^9$ zusammen mit X eine zusätzliche Kohlenstoff-Kohlenstoff-Bindung, $R^{10}$ Wasserstoff und $R^{11}$ und $R^{12}$ je Methyl bedeuten oder $R^9$ und $R^{11}$ zusammen Tetramethylen, $R^{10}$ und $R^{12}$ je Wasserstoff und X Methoxy bedeuten.

8. Verfahren gemäss Anspruch 7, dadurch gekennzeichnet, dass man eine Verbindung der in Anspruch 1 definierten Formel I herstellt, worin entweder $R^9$ und $R^{10}$ je Wasserstoff, $R^{11}$ und $R^{12}$ je Methyl und X Chlor, Hydroxy, Methoxy, Aethoxy, Propoxy, Acetoxy, 2-Hydroxyäthoxy oder 2-Methoxyäthoxy bedeuten oder $R^9$ zusammen mit X eine zusätzliche Kohlenstoff-Kohlenstoff-Bindung, $R^{10}$ Wasserstoff und $R^{11}$ und $R^{12}$ je Methyl bedeuten.

9. Verfahren gemäss Anspruch 8, dadurch gekennzeichnet, dass man eine Verbindung der Anspruch 1 definierten Formel I herstellt, worin $R^9$ und $R^{10}$ je Wasserstoff, $R^{11}$ und $R^{12}$ je Methyl und X Aethoxy, Acetoxy, 2-Hydroxyäthoxy oder 2-Methoxyäthoxy bedeuten.

10. Verfahren gemäss Anspruch 1, dadurch gekennzeichnet, dass man 2-[[[2-(2-Hydroxyäthoxy)-2-methylpropyl]thio]methyl]-4-methoxy-3-methyl-1-[5-(trifluormethyl)-2-benzimidazolyl]pyridinium-methansulfonat herstellt.

11. Verfahren gemäss Anspruch 1, dadurch gekennzeichnet, dass man intramolekular deprotonisiertes 2-[[[2-(2-Hydroxyäthoxy)-2-methylpropyl]thio]methyl]-4-methoxy-3-methyl-1-[5-(trifluormethyl)-2-benzimidazolyl]pyridiniumkation herstellt.

12. Verfahren gemäss Anspruch 1, dadurch gekennzeichnet, dass man 4-Methoxy-2-[[[2-(2-methoxyäthoxy)-2-methylpropyl]thio]methyl]-3-methyl-1-[5-(trifluormethyl)-2-benzimidazolyl]pyridinium-methansulfonat herstellt.

13. Verfahren gemäss Anspruch 1, dadurch gekennzeichnet, dass man intramolekular deprotonisiertes 4-Methoxy-2-[[[2-(2-methoxyäthoxy)-2-methylpropyl]thio]methyl]-3-methyl-1-[5-(trifluormethyl)-2-benzimidazolyl]pyridiniumkation herstellt.

14. Verfahren gemäss Anspruch 1, dadurch gekennzeichnet, dass man 2-[[(2-Acetoxy-2-methylpropyl)-thio]methyl]-4-methoxy-3-methyl-1-[5-(trifluormethyl)-2-benzimidazolyl]pyridinium-methansulfonat herstellt.

15. Verfahren gemäss Anspruch 1, dadurch gekennzeichnet, dass man 2-[[(2-Acetoxy-2-methylpropyl)-thio]methyl]-4-methoxy-3-methyl-1-[5-(trifluormethyl)-2-benzimidazolyl]pyridiniumchlorid herstellt.

16. Verfahren gemäss Anspruch 1, dadurch gekennzeichnet, dass man intramolekular deprotonisiertes 2-[[(2-Acetoxy-2-methylpropyl)thio]methyl]-4-methoxy-3-methyl-1-[5-(trifluormethyl)-2-benzimidazolyl]-pyridiniumkation herstellt.

17. Verfahren gemäss Anspruch 1, dadurch gekennzeichnet, dass man 2-[[(2-Aethoxy-2-methylpropyl)-thio]methyl]-4-methoxy-3-methyl-1-[5-(trifluormethyl)-2-benzimidazolyl]pyridinium-methansulfonat herstellt.

18. Verfahren gemäss Anspruch 1, dadurch gekennzeichnet, dass man 2-[[(2-Aethoxy-2-methylpropyl)-thio]methyl]-4-methoxy-3-methyl-1-[5-(trifluormethyl)-2-benzimidazolyl]pyridiniumchlorid herstellt.

19. Verfahren gemäss Anspruch 1, dadurch gekennzeichnet, dass man intramolekular deprotonisiertes 2-[[(2-Aethoxy-2-methylpropyl)thio]methyl]-4-methoxy-3-methyl-1-[5-(trifluormethyl)-2-benzimidazolyl]-pyridiniumkation herstellt.

20. Verfahren zur Herstellung von Arzneimitteln, insbesondere zur Bekämpfung von Ulcus ventriculi und/oder duodeni, dadurch gekennzeichnet, dass man eine oder mehrere Verbindungen der in Anspruch 1 definierten Formel I und gegebenenfalls einen oder mehrere andere therapeutisch wertvolle Stoffe zusammen mit einem oder mehreren therapeutisch inerten Excipientien in eine galenische Darreichungsform bringt.

21. Verwendung von Verbindungen der in Anspruch 1 definieten Formel I zur Herstellung von Arzneimitteln gegen Ulcus ventriculi und/oder duodeni.